(19)

**Europäisches Patentamt**
**European Patent Office**
**Office européen des brevets**

(11)     **EP 4 324 903 A1**

(12)                          **EUROPEAN PATENT APPLICATION**
                              published in accordance with Art. 153(4) EPC

(43) Date of publication:
**21.02.2024 Bulletin 2024/08**

(51) International Patent Classification (IPC):
***C12G 1/02*** *(2006.01)*

(21) Application number: **21936848.7**

(52) Cooperative Patent Classification (CPC):
**C12G 1/02**

(22) Date of filing: **14.04.2021**

(86) International application number:
**PCT/ES2021/070247**

(87) International publication number:
**WO 2022/219205 (20.10.2022 Gazette 2022/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Talleres Ruiz S.A.**
**26006 Logroño (ES)**

(72) Inventors:
• **SÁENZ- DÍEZ MURO, Juan Carlos**
  **26004 LOGROÑO (ES)**
• **RUIZ CABEZA, Roberto**
  **26006 LOGROÑO (ES)**
• **MAMOLAR DOMENECH, Sergio**
  **26006 LOGROÑO (ES)**

• **CARVAJAL FALS, Hipólito Domingo**
  **26004 LOGROÑO (ES)**
• **SÁNCHEZ ROCA, Ángel**
  **26004 LOGROÑO (ES)**
• **CRESPO SARIOL, Harold**
  **26004 LOGROÑO (ES)**
• **JIMÉNEZ MACÍAS, Emilio**
  **26004 LOGROÑO (ES)**
• **BLANCO FERNÁNDEZ, Julio**
  **26004 LOGROÑO (ES)**
• **PÉREZ DE LA PARTE, Mª Mercedes**
  **26004 LOGROÑO (ES)**
• **NIÑO MARTÍN, Daniel**
  **26003 LOGROÑO (ES)**

(74) Representative: **ABG Intellectual Property Law,
S.L.**
**Avenida de Burgos, 16D**
**Edificio Euromor**
**28036 Madrid (ES)**

(54)     **METHOD AND DEVICE FOR CONTINUOUS MONITORING AND IMPROVED AUTOMATIC
         CONTROL OF TEMPERATURE, AND OF AERATION-OXYGENATION, OF THE PROCESS OF
         ALCOHOLIC FERMENTATION IN WINE BY MEANS OF ACOUSTIC EMISSION TECHNIQUES**

(57)     The present invention relates to a device for continuous monitoring and improved automatic control of temperature, and of aeration-oxygenation, of the process of alcoholic fermentation in wine by means of acoustic emission techniques (D1), of the type of devices that incorporate means for tracking and controlling alcoholic fermentation in a self-emptying fermentation tank (0), and that mainly consists of: a. an instrumentation subsystem (1); b. an acoustic emission instrumentation subsystem (2); c. a gas processing and storage subsystem for the gases of air, $CO_2$, $N_2$ or $O_2$ (3); d. a control subsystem (4); and a method (P1) that uses the system (D1).

EP 4 324 903 A1

# FIG.01

**Description**

**Object and field of the art to which the invention belongs**

The invention relates to a method and device for continuous ("on-line", i.e., real-time) monitoring and automatic control of temperature of the process of alcoholic fermentation of grape must or of other sugary liquids using acoustic emission techniques in a fermentation tank, for the purpose of obtaining an improved automated control of temperature and of aeration-oxygenation in alcoholic fermentation.

Wine can only be obtained by means of alcoholic fermentation of grape must, this being the most important step in wine-making. In alcoholic fermentation (an anaerobic process), sugar from must is converted by means of anaerobic fermentation yeasts (e.g., *Saccharomyces cerevisiae,* which are capable of performing complete fermentation in the absence of oxygen; however, oxygen is a key parameter in the process of fermentation since yeasts need it during their growth stage) into alcohol (fundamentally ethanol) and carbon dioxide ($CO_2$), and a large amount of thermal energy is also given off. As it is in a liquid medium, $CO_2$ is generated in the form of bubbles which move up towards the surface. The release of the $CO_2$ is usually turbulent and causes the sensation of boiling, hence the name "fermentation", from the Latin expression *fervere.*

The present invention proposes a novel method and device for the continuous monitoring of the kinetics of alcoholic fermentation by means of measuring the acoustic emission originating with the formation, movement (up towards the surface), and collision of $CO_2$ bubbles for the purpose of implementing in said process an improved, continuous, and automated control of temperature and of aeration or oxygenation.

The invention belongs to the field for producing wine, beer, and alcoholic beverages (distilleries), and more specifically to a method and device for improved and automated control of temperature, and of aeration or oxygenation, of alcoholic fermentation for the wine-growing field.

**General principles**

Industrial fermentation of wine

Fermentation refers to the phase of the wine-making process in which must is transformed into wine. In the case of wine, the yeasts responsible for wine-making are single-celled (microscopic) fungi which are naturally found in grape skins (generally in a layer in the form of a fine white powder covering the skin of the grapes and referred to as bloom).

To curb the appearance of undesirable bacteria and other fermentation-limiting organisms, the must is sometimes sterilized with sulfur dioxide ($SO_2$) before the process of fermentation. One of the reasons is to inhibit beforehand the work of lactic acid bacteria while there is sugar in the must, preventing at that time the "souring" of the wine.

*Alcoholic fermentation*

The making of wine comprises a first fermentation called alcoholic fermentation, in which sugars in grape must are transformed into alcohol. It is a turbulent process due to the production of a significant bubbling for a period of ten to fifteen days. The process is carried out in stainless steel bioreactors or fermentation tanks where, as a result of the action of the different yeasts, the sugars are converted to ethyl alcohol (during the first few days of spontaneous fermentation, a series of species of yeast is produced until *Saccharomyces cerevisiae* wins out over the others due to the presence of ethyl alcohol, thereby being responsible for ending the process). Said bubbling favors the solid parts of the grapes (skins) rising up to the surface of the vat, forming a floating layer in the upper part thereof called the "cap". In the production of red wines, this cap imparts to the must an enormous quantity of substances contained in the skins (tannins, anthocyanins and flavonoids) as a result of which red wine acquires its characteristic reddish color and aromas. To favor the extraction of said substances existing in the skins and the mixing thereof with the must that is being fermented, it is necessary to break, soak, move, and, if it is even possible, submerge said cap, preventing it from becoming solid, and the method most widely used by wineries is the method known as "pumping over", which consists of moving the must from the lower part of the vat to the upper part, an operation that is performed several times a day (pumping over is furthermore necessary in the yeast multiplication stage since it aerates the must, but it does cause a loss of aromas). During the natural process of fermentation, it is possible to soften and guarantee an optimal quality of the wine, since alcohol acts as a barrier against possible substances that could negatively influence and damage the properties of the wine. Oxygen, acidity, as well as the presence of different substances, can trigger major impediments in the process of fermentation by hindering the work of the yeasts. For this reason, during alcoholic fermentation, a meticulous control of the different variables that affect the development of the process and impede the correct evolution of the yeasts must be carried out. Alcoholic fermentation is carried out with systems for the automatic control of temperature.

*Malolactic fermentation*

Once the first fermentation is finished, where all the sugar present in the grapes has been transformed and the $SO_2$ has been combined with other molecules, the next step is a second fermentation called malolactic fermentation, which can develop spontaneously. Malolactic fermentation of wine consists of the transformation of malic acid into lactic acid. Malic acid is found naturally in grapes and, by transforming it into lactic acid, a decrease in the total acidity of the wine is generated. This type of fermentation is generally carried out in red wines. This is because in white and rosé wines, it would cause a loss of acidity and freshness (two of the most important characteristics in wines of this type) because they are young wines. It is carried out in barrels, in stainless steel and concrete tanks (which have greater temperature stability in the optimal range of malolactic fermentation, i.e., from 19 to 23°C). In general, malolactic fermentation is carried out without systems for the automatic control of temperature.

Acoustic emission

Acoustic emission (AE), as it is currently known, is the propagation of mechanical waves in a solid caused by breakage or fracture of the material. When a material breaks, the material emits energy in the form of transient elastic waves that propagate through the material and can be detected by means of different types of sensors, such as piezoelectric sensors. The properties of AE can be a frequency of 10 kHz to 1 MHz and with an amplitude of the order of nm. AE is the kind of phenomenon that generates transient elastic waves by the rapid release of energy from localized sources, or transient waves generated in this way. Conventional sources of AE are deformation processes related to defects such as crack generation and propagation, material deformations, detachment of the aggregate from the matrix, contractions or expansions due to setting or temperature variations, etc. AE originates from the stress field created within the material. Therefore, if there are no variations in the stress field, AE does not occur.

**Review of the closest prior art**

*Tracking and characterization of the process of alcoholic fermentation*

Tracking the kinetics of alcoholic fermentation is done by controlling the fermentation temperature and measuring the amount of residual sugar and ethanol formed in the fermenting must; but the measurement of the density or the amount of $CO_2$ released are more practical tracking parameters to follow its evolution in order to immediately detect any disturbances occurring in the fermentation (Ribéreau-Gayon *et al.,* 2006a).

Fermentation temperature is one of the most important factors involved in the development of alcoholic fermentation. It is measured automatically and continuously by means of specific temperature sensors (Flanzy, 2003; Ribéreau-Gayon *et al.,* 2006a; Hidalgo, 2011). The temperature range (minimum-maximum in the fermentation tank) is 20-32°C for red wine and 18-20°C for white wine. In order for the biological reactor to function correctly, it must not exceed any of the aforementioned limit values. Below the minimum temperature, microbial kinetics are inhibited. Yeast reproduction is too slow and the fermenter does not "startup". Above 32°C, in addition to reducing the cellular activity of the microorganisms, there can be serious organoleptic deviations. In other words, the cap is susceptible to being affected by undesirable thermophilic bacteria, transmitting unpleasant odors to the wine.

However, the measurement of the rest of the parameters in each of the fermentation tanks of the winery is of great difficulty or involves tedious techniques that are impractical for tracking alcoholic fermentation in a winery (Cenzano *et al.,* 2003; Ribéreau-Gayon *et al.,* 2006a; OIV, 2015a). Therefore, conventional supervision of the kinetics of alcoholic fermentation has been carried out by manually measuring once or twice a day the density (graduated mustimeter, densimeter, or hydrometer), this being an approximate measurement of the main components of the fermented must; as fermentation progresses, the consumption of sugars causes a decrease in density, which is accentuated by the subsequent formation of ethanol (Ribéreau-Gayon *et al.* 2006a). This measurement does not require any conversion for its interpretation; it only needs a correction according to the temperature of the sample during its measurement.

The representation of density values versus time allows the winemaker to evaluate fermentation kinetics. Fermentation is complete when the density is between 0.990 and 0.995 g/mL depending on the final ethanol content and the amount of extracted constituents.

One of the most common methods to estimate the alcoholic strength of a wine is through its density; winemakers measure the density of the must before fermentation, and once the yeast has done its job, they measure again. The first result is called original gravity (OG) and the second result is called final gravity (FG). The number that results from subtracting the original gravity from the final gravity shows the amount of $CO_2$ that escaped from the fermentation tank. This amount is multiplied by the grams of ethyl alcohol gained per gram of $CO_2$ which is a constant 1.05 grams, and the result is the weight of alcohol in the fermenter, which is referred to as the mass of alcohol in solution. Once the results of these measurements are available, the alcohol by volume (ABV) percentage is calculated, being obtained by dividing the mass of alcohol in solution by the final gravity.

The completion of alcoholic fermentation is verified chemically by measuring the concentration of sugars using the Clinitest method, the content of which should be less than 2-3 g/L in dry or still wines (Ribéreau-Gayon *et al.,* 2006a; Hidalgo, 2011).

An upward increase in the probable alcoholic strength due to the effects of climate change can be seen in today's wine industry. Excess sugars in the must result in increased problems in fermentation management, while the increase in the final alcoholic strength leads to problems in marketing these wines. The main metabolic pathways that give rise to $CO_2$ production are respiration (no alcohol is produced) and fermentation (Morales, *et al.,* 2015).

In non-patent literature, a laboratory-level improvement in conventional alcoholic fermentation is known that aims to reduce the alcoholic strength of wine by means of ventilated fermentations with mixtures of air and nitrogen, allowing the yeast to perform respiration by providing the necessary oxygen during the first stages of the process (hours), such that a part of the sugars in the must undergoes respiration instead of fermentation; subsequently, the oxygen supply must be stopped in order to continue with the conventional process of fermentation; an excess presence of oxygen causes a reduced quality of the wine. On the contrary, there are no known devices and methods in fermentation tanks (real industrial-scale bioreactors) for oxygenation or aeration to allow yeasts to perform respiration (González *et al.,* 2013).

In the closest prior art patent literature, different types of devices and methods which allow the oxygenation or aeration of wine can be found. Within this type are, among others, the invention patent entitled "Method for fermenting must, and fermentation vat" (ES2396676B1, 25.02.2013), which proposes a method and a device for introducing a mixture of $CO_2$ gas and external air in a fermentation vat, said mixture being ejected into the must below the layer of skins, generating bubbles formed by $CO_2$ gas and air which, in their upward movement towards the surface, cause the breakage and movement of said layer of skins. In no case is respiration described since the sole purpose is to carry out fermentation.

*Control of the process of alcoholic fermentation*

In non-patent literature, one of the most widely studied improvements in the wine-growing field for the purpose of obtaining a higher quality in wine is the control of continuous alcoholic fermentation using to that end the control system theory; the problem from the viewpoint of systems engineering is highly non-linear and oscillatory, because microorganisms, e.g., *Saccharomyces cerevisiae, Zymomonas mobilis,* are involved in the process, and they exhibit behavior with high kinetic complexities. Reference problems (benchmarks) for processes of continuous fermentation using models with two state variables: biomass and substrate, and growth rate equations coupling both variables, are known to be used; said models thereby have high nonlinearities. The complex dynamics of the fermentation tanks or bioreactors are also investigated for the design of different stabilizing, non-linear control techniques for relatively simple processes of continuous fermentation, using for this purpose linear methods as a reference for non-linear methods. The control of bioreactors has been studied by many investigators, including those schemes based on adaptable control, optimal control and control using artificial intelligence (AI) techniques. However, investigations in this field are still in a latent state and an automatic method for optimized control which can be implemented in fermentation tanks (real industrial-scale bioreactors) with a high performance is not available in the current state of the art (Onder *et al.,* 1998; Sing and Xiao, 1998; Aguilar *et al.,* 2001; Hodge and Nazmul, 2002). As a result, most industrial wineries today only perform automatic control of the temperature during the process of fermentation through cooled liquid circulated through the external jacket of the alcoholic fermentation tank.

In the closest prior art patent literature, different types of devices and methods for the monitoring (measuring the temperature, measuring the level, measuring/calculating the density or some other variable) and continuous automated control (control of the temperature or some other variable, control of pumping over, etc.) of a process of alcoholic fermentation can be found.

Devices and methods based on the direct measurement of the fermentation temperature and the estimate by means of calculating the sugar and alcohol content from the indirect measurement of the density of the fermenting matter are known. This type includes, among many others, the patent document entitled "Process for monitoring and regulation of a fermentation process" (EP1270716A1, 02.01.2003), which proposes a device for the continuous monitoring and control of fermentation comprising a temperature sensor (thermal resistor) and a differential pressure sensor (piezoresistor) measuring at two different heights in the fermentation tank; the density at a certain temperature is calculated from the pressure differences measured at the measurement points; regulation of the temperature of the fermenting matter can furthermore be performed in the fermentation tank by means of the opening/closing of the passage of a cooled liquid going through a cooling plate located inside the tank.

Devices and methods which are based on the continuous direct measurement of alcohol in the fermenting matter are known. This type includes, among many others, the patent document entitled "Ethanol sensor for computerized fermentation control" (CA2011297A1, 01.09.1991), which proposes an ethanol sensor controlled by diffusion; the computer can automatically control the temperature and other process parameters for the fermenting matter.

Devices and methods that measure or determine the amount of $CO_2$ exiting the fermentation tank are also known. This type includes, among many others, the invention patent document entitled "Automatic fermentation provided with a control system" (ES2330242T3, 11.10.2006), which proposes a method of supervising a process of fermentation in a fermentation tank, where the amount of gas exiting said fermentation tank is determined and the temperature of the exiting gas is measured right above a plug arranged in the tap hole of the fermentation tank, in the plug of the fermentation tank, or at the end of the side of the fermenting matter of a fermentation airlock which is located in the tap hole. This type also includes the invention patent document entitled "System for tracking alcoholic fermentation" (ES2241457B1, 16.10.2005), which proposes a method for the tracking and control of alcoholic fermentation characterized by the continuous measurement of the weight loss of the fermenting matter due to the release of $CO_2$.

Summary of the State of the Art

During alcoholic fermentation, the fermentation mass (two phases can be distinguished inside the alcoholic fermentation tank: a liquid phase made up of the fermenting must and a solid phase known as "cap" (made up of the skin of the grape) gradually gains in alcoholic strength as the following fundamental parameters are modified:

- fermentation temperature (tend to increase), which is controlled to be constant (isothermal fermentation) or within a range (temperature-controlled fermentation), e.g., in red wines at 25°C or 22-26°C, respectively;
- volume (remains virtually constant);
- mass (decreases due to the loss of $CO_2$);
- density (decreases);
- total polyphenols (tend to decrease);
- anthocyanins (tend to decrease);
- organoleptic parameters (checked by means of sensory analysis);
- others (viscosity, refractive index, etc.).

When the fermentation ends (after around 10-14 days), the primary wine is transferred from the alcoholic fermentation tank to the malolactic fermentation tank for malolactic fermentation to take place, but the cap is not. The cap is transferred to the pneumatic press, from which a second-class wine will be obtained.

The automated method that is most widely used to perform the automated tracking of alcoholic fermentation consists of calculating the density of the liquid (which decreases due to the effect of the loss of mass due to the release of $CO_2$), by measuring the pressure and temperature difference at two points at different heights (the lower part and upper part of the cylindrical base body of the fermentation tank); it should be taken into account that most control systems for controlling alcoholic fermentation try to maintain the fermentation temperature at a value or within a range pre-established by the wine expert. Methods of measuring the amount of $CO_2$ given off by calculating, or in some cases by measuring, the weight loss of the fermenting matter, or in other cases by measuring the gas generated in real time by means of flowmeters, are also used.

The most advanced automated tracking systems try to detect the fermentation speed (g $CO_2$/l/h) and, therefore, the activity of the yeasts, this being a parameter that is as important as the temperature; to that end, they measure the amount, by weight or by volume, of $CO_2$ that is produced per unit of time for the purpose of controlling the fermentation temperature (Rib6reau-Gayon *et al.,* 2006a). The speed of alcoholic fermentation is proportional to the speed of generation of $CO_2$ (Perret *et al.,* 1995).

Another approach consists of creating a model of the process of alcoholic fermentation. Different calculations relating to the time, temperature, and alcohol, and the concentrations of sugar are used to predict the fermentation behavior, particularly the risk of the fermentation being stopped (Bovée *et al.,* 1990)

Finally, these rules of the control system must be adapted to the particular needs of each fermentation tank in terms of quantifiable data and according to the experience of the wine expert. A highly advanced, automated control system that seeks to optimize alcoholic fermentation in wine-making would have to use artificial intelligence in order to take into account the actual experience of the wine expert (Grenier *et al.,* 1990).

**Non-patent literature references**

- Aguilar, R., et al. Robust PI2 controller for continuous bioreactors. Process Biochemistry 36 (2001) 1007-1013.
- Bovée J.P., Blouin L, Marión, J.M. and Strehaiano, P. (1990) In Actualités Enologiques 90 (Ed. P. Ribéreau-Gayon and A. Lonvaud). Dunod, Paris, pp. 281-286.
- Cenzano, JM., Vicente, AM., Tejero, GM. (2003) Análisis de vinos, mostos y alcoholes. Antonio Madrid Vicente Ediciones and Ediciones Mundi-Prensa, Madrid.
- Flanzy, C. (2003) Enologia: fundamentos cientificos y tecnológicos, 2a. A. VICENTE EDICIONES and EDICIONES MUNDI-PRENSA, Madrid.
- González R., Quirós M., Morales R. (2013) Yeast respiration of sugars by non-Saccharomyces yeast species: a promising and barely explored approach to lowering alcohol content of wines. Trends Food Sci Technol; 29: 55-61.
- Grenier P., Feuilloley, P. and Sablayrolles, J.M. (1990). In Actualités Enologiques 89, (Ed. P. Ribéreau-Gayon and A. Lonvaud). Dunod, Paris, pp. 521-526.
- Hidalgo, J. (2011) Tratado de Enologia, Tomo 1, 2a. Mundi-Prensa, Madrid.
- Hodge, D. and Nazmul, M. Nonlinear MPC for recombinant ZM fedbatch ethanol fermentation. IFAC World Congress, 2002.
- Morales, P., et al. (2015) Levaduras no Saccharomyces como herramientas para controlar el grado alcohólico de los vinos: importancia del oxigeno y la respiración. Instituto de Ciencias de la Vid y del Vino, (CSIC - Universidad de La Rioja - La Rioja Government), Logroño. Available at: http://www.acenologia.com/cienciaytecnologia/no_saccharomyces_y_oxigeno_cie nc0515.htm
- OIV (2015) International Organization of Vine and Wine. Available at: http://www.oiv.int/es/la-organizacion-internacional-de-la-vina-y-el-vino.
- Onder, M., et al. Neural Network Assisted Nonlinear Controller for a Bioreactor Bogazici. Istanbul University, 1998.
- Ribéreau-Gayon, P., Dubourdieu, D., Donéche, B., Lonvaud, A. (2006a) Handbook of Enology - Volume 1 - The Microbiology of Wine and Vinifications, 2nd ed. John Wiley & Sons, Chichester.
- Ribéreau-Gayon, P., Glories, Y., Maujean, A., Dubourdieu, D. (2006b) Handbook of Enology - - Volume 2 - The Chemistry of Wine Stabilization and Treatments, 2nd ed. John Wiley & Sons, Chichester.
- Sing, K. and Xiao, D. Simple procedure for operating a class of continuous fermentation process at the optimal steady state production. Biochemical Engineering Journal. Vol. 1, 1997.

**Technical problem considered**

The devices and methods known in the prior art have a technical or problematic limitation in closed vat fermentation tanks, in which red wines are made without the need for pumping over (by means of injecting the $CO_2$ released during the alcoholic fermentation and aeration of the process of fermentation by means of controlled oxygenation), which is fundamentally focused on the following aspects:

X Systems that perform the automated tracking of alcoholic fermentation, based on the calculation of the density of the fermentation must by measuring the pressure and temperature difference with sensors located at different heights in the fermentation tank, are not reliable in closed tanks due to the error in the measurement ($\pm$ 3%, El Haloui *et al.* 1988) caused by the pressure produced by the $CO_2$ released in the upper part of the tank.

X Advanced automated tracking systems try to detect the fermentation speed by measuring the amount, by weight or by volume, of $CO_2$ that is produced per unit of time; but they are only capable of measuring released $CO_2$, i.e., once the saturation occurs in the fermentation must which passes into the air in gas form (it must be taken into account that the production of ethanol starts before $CO_2$ is released), therefore they have an initial error ($\pm$ 4%, El Haloui *et al.* 1988) and a delay time in the detection of the fermentation speed, which error is transferred to the system for the automatic control of the fermentation temperature.

X The most advanced automated tracking systems use a model of the process of alcoholic fermentation that has a modeling system with highly non-linear and oscillatory dynamics that are very hard to control (because various microorganisms (yeasts, molds, and bacteria) exhibiting a behavior with high kinetic complexities are involved in the process); therefore, they cannot be implemented with a high performance in fermentation tanks (large volume bioreactors) requiring periodic supervision of wine experts; furthermore, they use sensors which have certain complexity and technical ostentation in addition to problems relating to cleaning.

X There are known improvements in methods of aeration of the process of fermentation by means of controlled oxygenation (e.g., providing a continuous flow rate of 8 mg/1 of oxygen) for the purpose of preventing having to perform the mechanical pumping over, which may cause a loss of aromas in the case of excessive aeration, but they suffer from supplying an estimated flow rate of oxygen that differs from the exact flow rate required by yeasts depending on their actual phase of multiplication, causing deficiencies in aeration (causing fermentation to slow down, and in the worst case scenario, to stop) or excessive aeration (obtaining more acetic acid that what is acceptable). Furthermore, the known systems do not allow the yeasts to perform respiration in fermentation tanks (real industrial-scale bioreactors), but rather they seek to exclusively perform fermentation. It is therefore a sought-after need to find in fermentation tanks (real industrial-scale bioreactors) the working conditions in which the extraordinary supply of oxygen, which is necessary for the respiration and growth of the yeasts in fermentation, to be rapidly consumed by the yeasts, such that the oxygen that is effectively dissolved in the fermenting must is virtually nil (Morales *et al.,* 2015).

**Technical advantage provided by the invention**

The method and device for continuous monitoring and improved automatic control of temperature, and of aeration-oxygenation, of the process of alcoholic fermentation (AF) in wine by means of acoustic emission techniques, proposed by the invention, is preferably, though in a non-limiting manner, applied in self-emptying closed-vat alcoholic fermentation tanks (batch-type biological reactor) or any other fermentation tank in the current state of the art (preferably made of food-grade stainless steel with European Conformity (CE) or other similar international standards) in which red wines are made (also valid for rosé and white wines) without the need for (mechanical) pumping over and which allow the measurement of the released $CO_2$ flow, and which generally consist of three parts: an upper part referred to as a torispherical head, having high resistance to manometric pressure; a cylindrical base body and a lower part referred to as toriconical end, which is based on the ease of collecting the "cap", or any other form of fermentation tank in the current state of the art.

No modification of an existing fermentation tank is required for the implementation of the system of tracking and control of AF claimed by the invention, i.e., the invention is intended to be applied both in new tanks and in existing tanks.

The present invention solves in a fully satisfactory manner the drawbacks set forth above in each and every one the different aspects discussed, where the technical advantage provided by the invention which provides a method and means for being able to carry out the method using acoustic emission techniques is that it allows an improved estimate (without the need to use empirical approaches) of the typical parameters of AF from the following on-line measurements taken in the fermentation tank (by means of automated measurement sensors): pressure and temperature at two points of the fermentation must, pressure and temperature of the released $CO_2$, acoustic emission of $CO_2$ in dissolution and released $CO_2$ flow, which allow knowing the fermentation kinetics very accurately for the purpose of carrying out a control of the optimal fermentation temperature, as well as a control of the optimal aeration or oxygenation, by means of acoustic emission techniques.

The invention proposes, with respect to and controls of temperature of AF, of aeration or oxygenation known in the state of the art, measuring the acoustic emission of $CO_2$ in dissolution and in a released $CO_2$ flow which allows knowing at an early time the evolution of fermentation kinetics (speed) as it is proportional to the $CO_2$ generation speed, implementing an optimal control of temperature, aeration or oxygenation by means of acoustic emission techniques which have been found to achieve, for a maximum generated $CO_2$ speed of 0.81 g of $CO_2$/l/h, a reduction of 16.25%* (3.25% per degree of hysteresis) in the duration of fermentation with respect to the mean of isothermal controls of temperature in red wine (22°C-26°C), simultaneously obtaining an increase in wine quality of 5-10%* in the total polyphenol index (TPI) which causes a qualitative leap in terms of organoleptic parameters (proven in tasting sessions by means of sensory analysis).

(*) Results obtained by UNIVERSIDAD DE LA RIOJA during the 2018 and 2019 grape harvests. A campaign of *in situ* measurements in the must obtained in six conventional red wine fermentation tanks and in six tanks with the system claimed in the present invention was carried out in each grape harvest.

Brief Description of the Figures

To complement the description and for the purpose of helping to better understand the features of the invention, a set of illustrative and non-limiting figures as well as a glossary with the references used in the figures along with a description of each reference are attached as an integral part of said description.

*Glossary of references and description thereof*

**(0) Self-emptying fermentation tank, or any other type of fermentation tank, any one from the prior art;**

(01)      Ceiling; upper part of the casing of the tank;
(02)      Collar; cylindrical part of the casing of the tank formed by vertical cylinders;
(03)      Cone or bottom; lower part of the casing of the tank;

(continued)

(04)    Cone disc or bottom disc; lower part of the casing of the tank in the flat part in which the elements facilitating the emptying of the tank (blade-geared motor) are anchored;

(05)    Jacket; metal casing outside the collar which creates a chamber between the jacket and the collar where forced water internally circulates to control the temperature of the tank;

(06)    Pumping over and cleaning tube; tube which ends in the upper part of the tank in the dome which is used for tank cleaning and disinfection operations by means of recirculation in a closed circuit by connecting a pump from the total outlet;

(07)    Door; opening through which the door is moved by means of a mechanism and through which the bled pulp tank is emptied;

(08)    Blade-geared motor; set of mechanical elements which, as a result of the internal blade of the tank and the movement provided by the geared motor, facilitates the dry pulp emptying operation;

(09)    Total outlet; valve placed flush with the bottom of the cone disc in order to completely empty the tank of liquid;

(010)   Dome; last upper part of the tank with a cylindrical geometry containing the upper door by means of locks;

(011)   Vent valve; valve placed in the upper part of the tank in the dome element which, during filling or emptying operations of the liquid in the storage tank, prevents pressurizing/depressurizing action by means of air inlet or outlet;

(012)   Level gauge; element for reading the filling level of the tank;

(013)   Self-cleaning homogeneous mixer-bleeder system consisting of a set of screens and a bleed manifold;

(014)   Bleed valve; external connection valve of the bleed manifold (015) through which the must is extracted;

(015)   Bleed manifold; pipe outside the tank which joins all the bleed assemblies to concentrate all the must output in the bleed valve (014);

**(D1) Device for continuous monitoring and improved automatic control of temperature, and of aeration-oxygenation, of the process of alcoholic fermentation in wine by means of acoustic emission techniques, object of the invention;** comprises four subsystems:

-    Instrumentation subsystem (1);
-    Acoustic emission instrumentation subsystem (2);
-    Gas processing and storage subsystem for the gases of air, $CO_2$, $N_2$ or $O_2$ (3);
-    Control subsystem (4);

**(0, D1) Fermentation tank (0) incorporated in the invention (D1);**

**(1) Instrumentation subsystem of the device (D1), object of the invention;**

(10)    Torispherical head pressure sensor; this sensor measures the pressure (P) of $CO_2$ or other gases: air or $N_2$; it is combined with sensors (13, 15) to eliminate calculation error of the density (D) of the fermentation must;

(11)    Torispherical head temperature sensor; this sensor measures the temperature (T) of $CO_2$ or other gases: air or $N_2$;

(12)    Torispherical head flowmeter sensor; this sensor measures the flow rate (F) of suctioned $CO_2$ or other gases: injected air or $N_2$;

(13)    Upper cylindrical body pressure sensor; this sensor measures the pressure (P) in the liquid phase of the fermentation must below the area of the "cap"; combined with the sensor (15) and, by eliminating error by means of the sensor (10), the density (D) of the must and the level of the free surface are calculated;

(14)    Upper cylindrical body temperature sensor; this sensor measures the temperature (T) in the fermentation must below the area of the "cap";

(15)    Lower cylindrical body pressure sensor; this sensor measures the pressure (P) in the liquid phase of the fermentation must above the area of solids sunken to the bottom; combined with the sensor (13) and, by eliminating error by means of the sensor (10), the density (D) of the must and the level of the free surface are calculated;

(continued)

| | |
|---|---|
| (16) | Lower cylindrical body temperature sensor; this sensor measures the temperature (T) in the liquid phase of the fermentation must above the area of solids sunken to the bottom; |
| (17) | $CO_2$ or $N_2$ injection flowmeter sensor; this sensor measures the flow rate (F) of injected $CO_2$ or $N_2$; |
| (18) | Aeration or oxygenation flowmeter sensor; this sensor measures the flow rate (F) of injected air or $O_2$; |

**(2) Acoustic emission instrumentation subsystem of the device (D1), object of the invention;**

| | |
|---|---|
| (20) | Omnidirectional hydrophone sensor; this sensor measures the acoustic emission (A) produced within the fermentation must; |
| (21) | Analog signal conditioner; this conditioner preamplifies (AS) the voltage of the hydrophone (20); |

**(3) Gas processing and storage subsystem for the gases of air, $CO_2$, $N_2$ or $O_2$ of the device (D1), object of the invention;**

| | |
|---|---|
| (111) | Aeration or oxygenation control valve; normally closed (NC); being a 2-way type seated globe valve having 2 positions that is pneumatically or electrically operated and controlled within the control of the system. It has the function of allowing the injection of $O_2$ or air; |
| (113) | Cooling control valve; normally closed (NC); being a 2-way type seated globe valve having 2 positions that is pneumatically or electrically operated and controlled within the control of the system. It has the function of allowing or preventing the passage of liquid coolant to the jackets (05) of the fermentation tank (0, D1). |
| (31) | Outlet or injection valve; normally closed (NC); being a 2-way type seated globe valve having 2 positions that is pneumatically or electrically operated and controlled within the control of the system; it has the function of allowing the capturing or injection of $CO_2$ or other gases: air or $N_2$; |
| (32) | Inerting control valve; this is a valve located in the upper part of the tank in the dome element (010) which allows the action of pressurizing the tank (0); incompatible with the vent valve (011), so the valve (32) replaces the valve (011); |
| (33) | Suction; suction pipe through which $CO_2$ is captured or other gases: air or $N_2$, are injected; |
| (34) | Delivery; delivery pipe through which pressurized $CO_2$ or other gases: air or $N_2$, is conducted; |
| (35) | Injection control valve; being a 2-way type seated globe valve having 2 positions that is pneumatically or electrically operated and controlled within the control of the system; it has the function of allowing the entry of $CO_2$ or other gases: air or $N_2$; |
| (36) | $CO_2$ injection collector; a pipe outside the tank which connects all the $CO_2$ injection elements with the inlet valve (035); |
| (310) | Selector valve for the capturing of $CO_2$ or air; being a 3-way type seated globe valve with 3 positions that is pneumatically or electrically operated and controlled within the control subsystem (4); it has the function of selecting the capturing of $CO_2$, or air; |
| (311) | Selector valve for the delivery of $CO_2$ or air; being 3-way type seated globe valve with 3 positions that is pneumatically or electrically operated and controlled within the control subsystem (4); it has the function of selecting the delivery of $CO_2$ or air; |
| (312) | Selector valve for the delivery of air; being 3-way type seated globe valve with 3 positions that is pneumatically or electrically operated and controlled within the control subsystem (4); it has the function of selecting the delivery of air; |
| (313) | Selector valve for the delivery of air or $O_2$; being a 3-way type seated globe valve with 3 positions that is pneumatically or electrically operated and controlled within the control subsystem (4); it has the function of selecting the delivery of air or $O_2$. |
| (314) | Trap filter tank; this tank prevents the entry of condensate into the compressors, protecting them from said condensate; |

(continued)

| | | |
|---|---|---|
| (315) | Compressor; having a design pressure of 10 bar according to ISO 8573-1 standard, class 0, in terms of the quality of air suitable for the food industry; | |
| (316) | Pressurized $CO_2$ or $N_2$ tank; being a pressurized buffer tank with a design pressure of 10 bar; | |
| (317) | Pressurized air tank; being a pressurized buffer tank with a design pressure of 10 bar; | |
| (318) | $N_2$ generator; being an $N_2$ generator for inerting the fermentation tank (0); | |
| (319) | Pressurized $N_2$ tank; being a pressurized buffer tank with a design pressure of 10 bar; | |
| (320) | Compressed $O_2$ cylinder; | |

**(4) Control subsystem of the device (D1), object of the invention;**

| | |
|---|---|
| (40) | Main control panel; |
| (401) | Programmable logic controller (PLC); |
| (402) | Human machine interface (HMI); |
| (41) | Slave panel; incorporating decentralized periphery modules that collect input signals of the instrumentation subsystems for sending data to the panel (40) by means of a single communications bus. |
| (42) | Secondary panel; incorporating solenoid valve modules that collect control data of the panel (40) by means of a single communications bus in order to act on the valves of the instrumentation subsystems. |

**(P1) Method for continuous monitoring and improved automatic control of temperature of the process of alcoholic fermentation in wine by means of acoustic emission techniques, object of the invention;**

| | | |
|---|---|---|
| (P0) | PLC program (401); | |
| (P10) | STEP "a" | Respiration with air; |
| (P11) | SUB-STEP | Injecting air in the fermentation tank (0, D1); |
| (P12) | SUB-STEP | Suctioning $CO_2$ + injecting air in the fermentation tank (0, D1); |
| (P13) | SUB-STEP | Suctioning $CO_2$ + injecting air and $CO_2$ in the fermentation tank; |
| (P100) | STEP "a.0" | Refilling the air tank (317); |
| (P20) | STEP "b" | Respiration with $O_2$; |
| (P21) | SUB-STEP | Injecting $O_2$ in the fermentation tank (0, D1); |
| (P22) | SUB-STEP | Suctioning $CO_2$ + injecting $O_2$ in the fermentation tank (0, D1); |
| (P23) | SUB-STEP | Suctioning $CO_2$ + injecting $O_2$ and $CO_2$ in the fermentation tank(0, D1); |
| (P30) | STEP "c" | Alcoholic fermentation plus aeration; |
| (P31) | SUB-STEP | Suctioning $CO_2$ + injecting $CO_2$ in the fermentation tank (0, D1); |
| (P32) | SUB-STEP | Suctioning $CO_2$ in the fermentation tank (0, D1); |
| (P40) | STEP "d" | Alcoholic fermentation plus oxygenation; |
| (P50) | STEP "e" | Inerting with $N_2$; |
| (P60) | STEP "f" | Normal stop; |
| (P70) | STEP "g" | Optimized control of temperature; |
| (P80) | STEP "h" | Optimized control of aeration or oxygenation; |
| (P90) | STEP "i" | Emergency stop; |
| (P101) | MODULE | For calibration; |
| (P102) | MODULE | For filtration. |

*Glossary of symbols used in Graphs of Control by Steps and Transitions (GRAFCET) and Piping and Instrumentation Diagrams (P&IDs).*

| | |
|---|---|
| P | relative pressure of the liquid phase of the fermentation must or of $CO_2$ or other gases: air or $N_2$; |
| T | temperature of the liquid phase of the fermentation must or of $CO_2$ or other gases: air or $N_2$; |
| F | flow or flow rate of the liquid phase of the fermentation must or of $CO_2$ or other gases: air or $N_2$; |
| D | density of the liquid phase of the fermentation must or of $CO_2$ or other gases: air or $N_2$; |
| A | acoustic emission of the liquid phase of the fermentation must or of $CO_2$ or other gases: air or $N_2$; |
| AS | Analog signal conditioner or preamplifier; |
| NA | Normally open contact; |
| NC | Normally closed contact; |
| Pa | NA push button, activation step "a"; |
| Pb | NA push button, activation step "b"; |
| Pc | NA push button, activation step "c"; |
| Pd | NA push button, activation step "d"; |
| Pe | NA push button, activation step "e"; |
| Pf | NA push button, activation step "f"; |
| ST | NA selector, activation step "g"; |
| SA/O | NA selector, activation step "h"; |
| PE | NC emergency push button; |
| P-E | Denied logic query of the NC emergency push button; |
| Pa* | Logic eq. = Pb+Pc+Pd+Pe+Pf; |
| Pb* | Logic eq. = Pa+Pc+Pd+Pe+Pf; |
| Pc* | Logic eq. = Pa+Pb+Pd+Pe+Pf; |
| Pd* | Logic eq. = Pa+Pb+Pc+Pe+Pf; |
| Pe* | Logic eq. = Pa+Pb+Pc+Pd+Pf; |
| Pf* | Logic eq. = Pa+Pb+Pc+Pd+Pe; |
| 3100 | Bit "0" for activation of position 1 of the selector valve for the capturing of $CO_2$ or air (310); |
| 3101 | Bit "1" for activation of position 2 of the selector valve for the capturing of $CO_2$ or air (310); |
| 3110 | Bit "0" for activation of position 1 of the selector valve for the delivery of $CO_2$ or air (311); |
| 3111 | Bit "1" for activation of position 2 of the selector valve for the delivery of $CO_2$ or air (311); |
| 3120 | Bit "0" for activation of position 1 of the selector valve for the delivery of air (312); |
| 3121 | Bit "1" for activation of position 2 of the selector valve for the delivery of air (312); |
| 3130 | Bit "0" for activation of position 1 of the selector valve for the delivery of air or $O_2$ (313); |
| 3131 | Bit "1" for activation of position 2 of the selector valve for the delivery of air or $O_2$ (313); |
| p316 | Pressure in $CO_2$ tank (316); |
| p316min | Minimum pressure in $CO_2$ tank (316); |
| p316max | Maximum pressure in $CO_2$ tank (316); |
| p317 | Pressure in air tank (317); |
| p317min | Minimum pressure in air tank (317); |
| p317max | Maximum pressure in air tank (317); |
| p319 | Pressure in $N_2$ tank (319); |
| p319min | Minimum pressure in $N_2$ tank (319); |
| p319max | Maximum pressure in $N_2$ tank (319); |
| p10min | Minimum pressure in pressure sensor (10); |
| p10max | Maximum pressure in pressure sensor (10); |
| f18 | Accumulated flow measured by the aeration or oxygenation flowmeter sensor (18); |
| fsetpoint | Accumulated flow setpoint for aeration or oxygenation; |

(continued)

| | |
|---|---|
| Tsetpoint | Temperature setpoint; |
| $\Delta$T | Temperature hysteresis; |
| $(ACO_2)$setpoint | Acoustic emission $CO_2$ setpoint; |
| $\Delta(ACO_2)$ | Acoustic emission hysteresis; |
| 111on = 1 | Command bit for activating the oxygenation control valve (111); |
| 111on = 0 | Command bit for deactivating the oxygenation control valve (111); |
| 31on = 1 | Command bit for activating the outlet or injection valve (31); |
| 31on = 0 | Command bit for deactivating the outlet or injection valve (31); |
| 35on =1 | Command bit for activating the injection control valve (35); |
| 35on =0 | Command bit for deactivating the injection control valve (35). |

*Glossary of symbols used in the acoustic emission techniques*

| | |
|---|---|
| $ACO_2$ | Acoustic emission caused by the $CO_2$ generated in respiration and alcoholic fermentation; |
| $dACO_2/dt$ | Speed of the acoustic emission of the $CO_2$ generated; |
| g $CO_2$/l/h | Speed of the alcoholic fermentation (amount, by weight or by volume, of $CO_2$ produced per unit of time); |
| (AMF) | Acoustic emission measured in the fermentation must; |
| (AAera) | Acoustic emission caused by noise from aeration; |
| $(AO_2)$ | Acoustic emission caused by noise from oxygenation; |
| $(AICO_2)$ | Acoustic emission caused by noise from injection of $CO_2$; |
| (AR) | Acoustic emission caused by noise from cooling; |
| (AO) | Acoustic emission caused by another noise. |

*Glossary of symbols used in the simple conditional logic equations*

| | |
|---|---|
| "If" | Conditional yes instruction; |
| () | Condition; |
| "AND" | Conditional instruction for logical multiplication); |
| "OR" | Conditional instruction for logical addition); |
| "then" | Assignment instruction; |
| {} | Logical operation. |

Figure 1 (FIG. 1) shows a 3D computer graphic of a fermentation tank (0), any one from the prior art, which incorporates a device for continuous monitoring and improved automatic control of temperature of the process of alcoholic fermentation in wine by means of acoustic emission techniques (D1), object of the invention, in which the implementation of the subsystems (1, 2, 3, and 4) comprised in same can be observed.

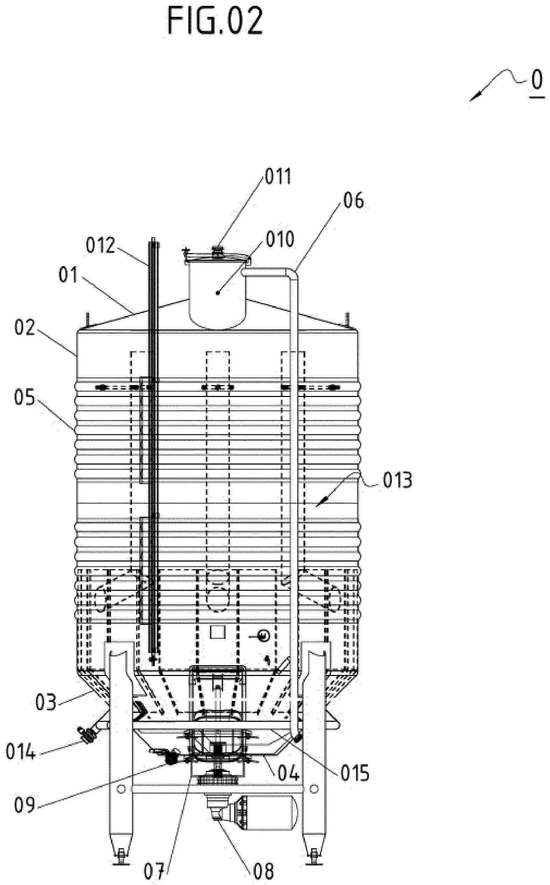

Figure 2 (FIG. 2) shows an elevational view of a self-emptying fermentation tank (0), any one from the prior art; in which the elements comprised in same can be observed.

Figure 3 (FIG. 3) shows a schematic view of the instrumentation subsystem (1) of the device (D1), object of the invention; in which the elements comprised in same can be observed.

Figure 4 (FIG. 4) shows a schematic view of the acoustic emission instrumentation subsystem (2) of the device (D1), object of the invention; in which the elements comprised in same can be observed.

Figure 5 (FIG. 5) shows an elevational view of the gas processing and storage subsystem for the gases of air, $CO_2$,

$N_2$, or $O_2$ (3) of the device (D1), object of the invention; in which the interconnecting elements with a self-emptying fermentation tank (0) can be observed.

Figure 6 (FIG. 6) shows a P&ID diagram of the gas processing and storage subsystem for the gases of air, $CO_2$, $N_2$, or $O_2$ (3) of the device (D1) in the operating mode: REFILLING AIR TANK (317).

Figure 7 (FIG. 7) shows a P&ID diagram of the gas processing and storage subsystem for the gases of air, $CO_2$, $N_2$, or $O_2$ (3) of the device (D1) in the operating mode: REFILLING $N_2$ TANK (318).

Figure 8 (FIG. 8) shows a P&ID diagram of the gas processing and storage subsystem for the gases of air, $CO_2$, $N_2$, or $O_2$ (3) of the device (D1) in the operating mode: INJECTING AIR IN THE FERMENTATION TANK (0, D1).

Figure 9 (FIG. 9) shows a P&ID diagram of the gas processing and storage subsystem for the gases of air, $CO_2$, $N_2$, or $O_2$ (3) of the device (D1) in the operating mode: SUCTIONING $CO_2$ + INJECTING AIR IN THE FERMENTATION TANK (0, D1).

Figure 10 (FIG. 10) shows a P&ID diagram of the gas processing and storage subsystem for the gases of air, $CO_2$, $N_2$, or $O_2$ (3) of the device (D1) in the operating mode: SUCTIONING $CO_2$ + INJECTING AIR and $CO_2$ IN THE FERMENTATION TANK (0, D1).

Figure 11 (FIG. 11) shows a P&ID diagram of the gas processing and storage subsystem for the gases of air, $CO_2$, $N_2$, or $O_2$ (3) of the device (D1) in the operating mode: SUCTIONING $CO_2$ + INJECTING $CO_2$ IN FERMENTATION TANK (0, D1).

Figure 12 (FIG. 12) shows a P&ID diagram of the gas processing and storage subsystem for the gases of air, $CO_2$, $N_2$, or $O_2$ (3) of the device (D1) in the operating mode: SUCTIONING $CO_2$ IN THE FERMENTATION TANK (0, D1).

Figure 13 (FIG. 13) shows a P&ID diagram of the gas processing and storage subsystem for the gases of air, $CO_2$, $N_2$, or $O_2$ (3) of the device (D1) in the operating mode: INJECTING $O_2$ IN THE FERMENTATION TANK (0, D1).

Figure 14 (FIG. 14) shows a P&ID diagram of the gas processing and storage subsystem for the gases of air, $CO_2$, $N_2$, or $O_2$ (3) of the device (D1) in the operating mode: SUCTIONING $CO_2$ + INJECTING $O_2$ IN THE FERMENTATION TANK (0, D1).

Figure 15 (FIG. 15) shows a P&ID diagram of the gas processing and storage subsystem for the gases of air, $CO_2$, $N_2$, or $O_2$ (3) of the device (D1) in the operating mode: SUCTIONING $CO_2$ + INJECTING $O_2$ and $CO_2$ IN THE FERMENTATION TANK (0, D1).

Figure 16 (FIG. 16) shows a P&ID diagram of the gas processing and storage subsystem for the gases of air, $CO_2$, $N_2$, or $O_2$ (3) of the device (D1) in the operating mode: INERTING BY MEANS OF $N_2$ IN THE FERMENTATION TANK (0, D1).

Figure 17 (FIG. 17) shows a GRAFCET diagram with the start block, which is cyclically executed, implemented in a PLC program (P0), installed in a PLC (401).

Figure 18 (FIG. 18) shows a GRAFCET diagram with STEP "a.0" of refilling the air tank (317) (P100) and STEP "a" of respiration with air (P10), implemented in a PLC program (P0), installed in a PLC (401).

Figure 19 (FIG. 19) shows a GRAFCET diagram with STEP "b" of respiration with $O_2$ (P20), implemented in a PLC program (P0), installed in a PLC (401).

Figure 20 (FIG. 20) shows a GRAFCET diagram with STEP "a.0" of refilling the air tank (317) (P100) and STEP "c" of alcoholic fermentation plus aeration (P30), implemented in a PLC program (P0), installed in a PLC (401).

Figure 21 (FIG. 21) shows a GRAFCET diagram with STEP "d" of alcoholic fermentation plus oxygenation (P40), implemented in a PLC program (P0), installed in a PLC (401).

Figure 22 (FIG. 22) shows a GRAFCET diagram with STEP "g" of optimized control of temperature (P70), implemented in a PLC program (P0), installed in a PLC (401).

Figure 23 (FIG. 23) shows a GRAFCET diagram with STEP "h" of optimized control of aeration or oxygenation (P80), implemented in a PLC program (P0), installed in a PLC (401).

**Detailed Description of the Invention and Detailed Disclosure of a Preferred Embodiment of the Invention**

A preferred embodiment of the invention, from among different possible alternatives, is described in detail by means of listing the components of the embodiment, as well as their functional relationship based on references to the figures, which have been included, in an illustrative and non-limiting manner, according to the principles of the claims. Reference is made to the figures, where necessary, in order to better understand what is shown therein.
Once the bunches of grape are destemmed (with the stem removed), the dense must (pulp or mesocarp, seeds, skins, and bloom) coming from the red grape, although it can also be rosé or even white, is sent to the self-emptying fermentation tank (0) provided with a device for continuous monitoring and improved automatic control of temperature, and of aeration-oxygenation, of the process of alcoholic fermentation in wine by means of acoustic emission techniques (D1), in order to proceed with alcoholic fermentation.
The invention proposes, with respect to and controls of temperature of AF, of aeration or oxygenation known in the state of the art, measuring the acoustic emission of $CO_2$ in dissolution and in a released $CO_2$ flow which allows knowing at an early time the evolution of fermentation kinetics (speed) as it is proportional to the $CO_2$ generation speed, implementing an optimal control of temperature, aeration or oxygenation by means of acoustic emission techniques which have been found to achieve, for a maximum generated $CO_2$ speed of 0.81 g of $CO_2$/l/h, a reduction of 16.25%* (3.25% per degree of hysteresis) in the duration of fermentation with respect to the mean of isothermal controls of temperature in red wine (22°C-26°C), simultaneously obtaining an increase in wine quality of 5-10%* in the total polyphenol index (TPI) which causes a qualitative leap in terms of organoleptic parameters (proven in tasting sessions by means of sensory analysis).
(*) Results obtained by UNIVERSIDAD DE LA RIOJA during the 2018 and 2019 grape harvests. A campaign of *in situ* measurements in the must obtained in six conventional red wine fermentation tanks and in six tanks with the system claimed in the present invention was carried out in each grape harvest.
Therefore, the invention seeks to perform continuous tracking of alcoholic fermentation in wine by means of acoustic emission techniques using the following subsystems comprised in the invention: - an instrumentation subsystem (1); - an acoustic emission instrumentation subsystem (2); - a gas processing and storage subsystem for the gases of air, $CO_2$, $N_2$ or $O_2$ (3) ; and - a control subsystem (4).
The invention proposes a device for continuous monitoring and improved automatic control of temperature of the process of alcoholic fermentation in wine by means of acoustic emission techniques (D1), of the type of devices that incorporate means for tracking and controlling alcoholic fermentation in a self-emptying fermentation tank (0), and is **characterized in that** the means for tracking and controlling comprise:

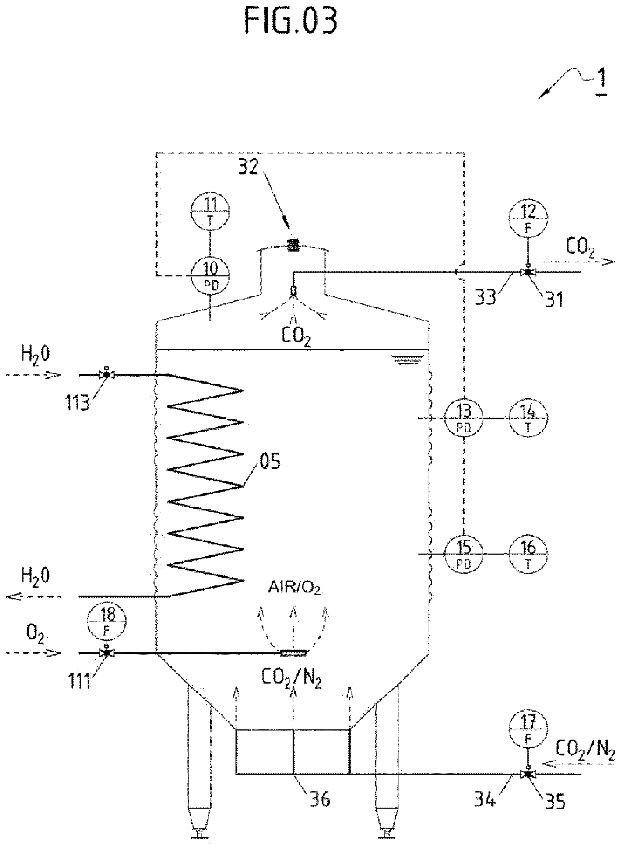

> a. an instrumentation subsystem (1) (Figure 03) which, by means of a plurality of sensors, measures and calculates a series of parameters in the fermentation must, containing in a non-limiting preferred embodiment:
> A set of sensors arranged in the self-emptying fermentation tank (0) in the torispherical head, these sensors being:
>
> - a torispherical head pressure sensor (10); this sensor measures the pressure (P) of $CO_2$ or other gases: air or $N_2$; it is combined with sensors (13, 15) to eliminate calculation error of the density (D) of the fermentation must; being of a flush membrane-type pressure sensor for red wines having a range of 0-160 mbar and for white wines having a range of 0-250 mbar;
> - a torispherical head temperature sensor (11); this sensor measures the temperature (T) of $CO_2$ or other gases: air or $N_2$; being of a PT100 probe type for measuring from 10 to 40°C;
> - a torispherical head flowmeter sensor (12); this sensor measures the flow rate (F) of suctioned $CO_2$ or other gases: injected air or $N_2$; having a range of 10-1000 l/min or for installations with many compressors and suctions of 10-2000 l/min.

A set of sensors arranged in the self-emptying fermentation tank (0) in the upper cylindrical body, these sensors being:

- an upper cylindrical body pressure sensor (13); this sensor measures the pressure (P) in the liquid phase of the fermentation must below the area of the "cap"; combined with the sensor (15) and, by eliminating error by means of the sensor (10), the density (D) of the must and the level of the free surface are calculated; being of a flush membrane-type pressure sensor having a range of 0-400 mbar for tanks up to 4 m in height and of 0-1 bar for tanks up to 10 m in height;

- an upper cylindrical body temperature sensor (14); this sensor measures the temperature (T) in the fermentation must below the area of the "cap"; being of a PT100 probe type for measuring from 10 to 40°C;

A set of sensors arranged in the self-emptying fermentation tank (0) in the lower cylindrical body, these sensors being:

- a lower cylindrical body pressure sensor (15); this sensor measures the pressure (P) in the liquid phase of the fermentation must above the area of solids sunken to the bottom; combined with the sensor (13) and, by eliminating error by means of the sensor (10), the density (D) of the must and the level of the free surface are calculated; being of a flush membrane-type pressure sensor having a range of 0-400 mbar for tanks up to 4 m in height and of 0-1 bar for tanks up to 10 m in height;
- a lower cylindrical body temperature sensor (16); this sensor measures the temperature (T) in the liquid phase of the fermentation must above the area of solids sunken to the bottom; being of a PT100 probe type for measuring from 10 to 40°C;
- an aeration or oxygenation flowmeter sensor (18); this sensor measures the flow rate (F) of injected air or $O_2$; having a range of 0-3000 l/min for $CO_2$ injection and a range of 0-10 1/min for $O_2$ injection.

Therefore, calculation of the density (D) of the fermentation must is performed based on measurements given by pressure sensors (13, 15). Since the tank is closed, the $CO_2$ released in the upper part thereof causes a pressure in the fermentation must which causes a calculation error of the density (D) of the fermentation must; this is due to the fact that Pascal's principle which states that "pressure applied to a point of a static incompressible fluid confined in a container is transmitted entirely to all the points of the fluid" is not complied with given that the fermentation must contains a fraction of $CO_2$ in dissolution, which is a compressible gas; error elimination is carried out based on the measurement given by the pressure sensor (10) which measures the pressure (P) of $CO_2$ or other gases. To that end, the invention proposes performing an initial calibration before starting alcoholic fermentation which consists of performing a first measurement by means of the three pressure sensors (13, 15, and 10) when there is still no $CO_2$ dissolved in the fermentation must (error-free measurement) and a second measurement by injecting $CO_2$ (measurement with error), opening the injection control valve (35) of the gas processing and storage subsystem (3) until the pressure sensor (10) indicates the same $CO_2$ pressure as the working pressure marked by the inerting control valve (32); the error produced in the pressure sensors (13, 15) (error = measurement with error - error-free measurement) is obtained with both measurements. In general, the pressure of the inerting control valve (32) is fixed for each type of tank, so calibration only needs to be performed once.

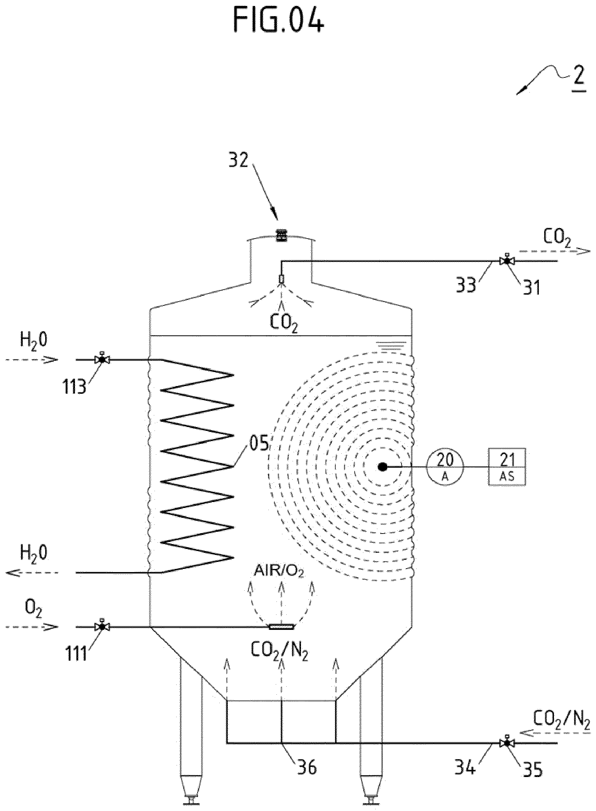

b. acoustic emission instrumentation subsystem (2) (Figure 04) which, by means of a set of sensors, measures the acoustic emission caused by the $CO_2$ generated during respiration and alcoholic fermentation in the fermentation must, containing in a non-limiting preferred embodiment:

- an omnidirectional-type hydrophone sensor (20); this sensor measures the acoustic emission (A) produced within the fermentation must; having a range of 1 Hz to 140 kHZ, of horizontal positioning and $\pm$2dB omnidirectional type with an operating temperature range of -2°C to +80°C, of the type that is submersible and resistant to wine must and acetic acid, and is arranged submerged in the fermentation must contained in the self-emptying fermentation tank (0);
- an analog signal conditioner (21); this conditioner preamplifies (AS) the voltage of the hydrophone (20); having an output DC voltage range of 0-10 V;

In this case, ultrasound signals are not processed, rather the vibro-acoustic emission (below the ultrasonic frequency range) generated by the carbon dioxide gas resulting from respiration and alcoholic fermentation is captured. The effects of temperature on the alcoholic fermentation range do not significantly affect measurements.

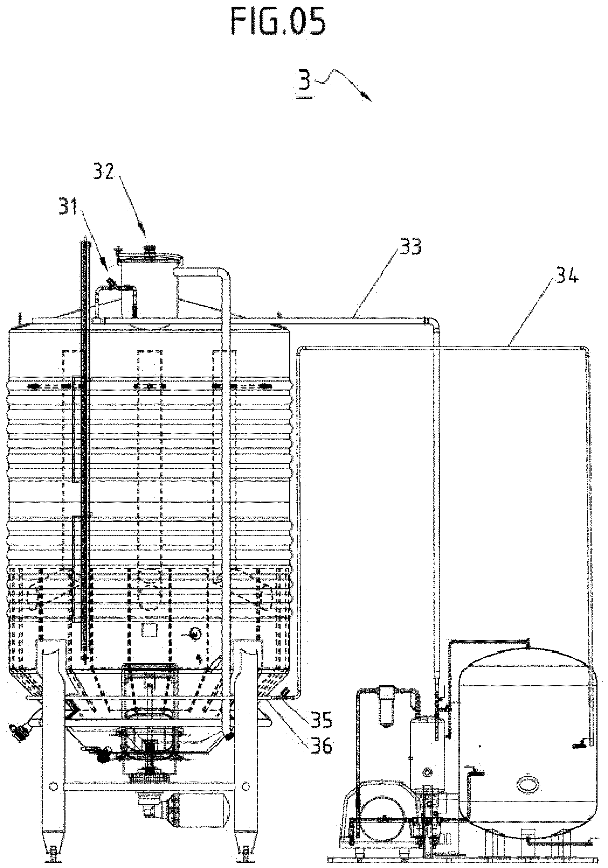

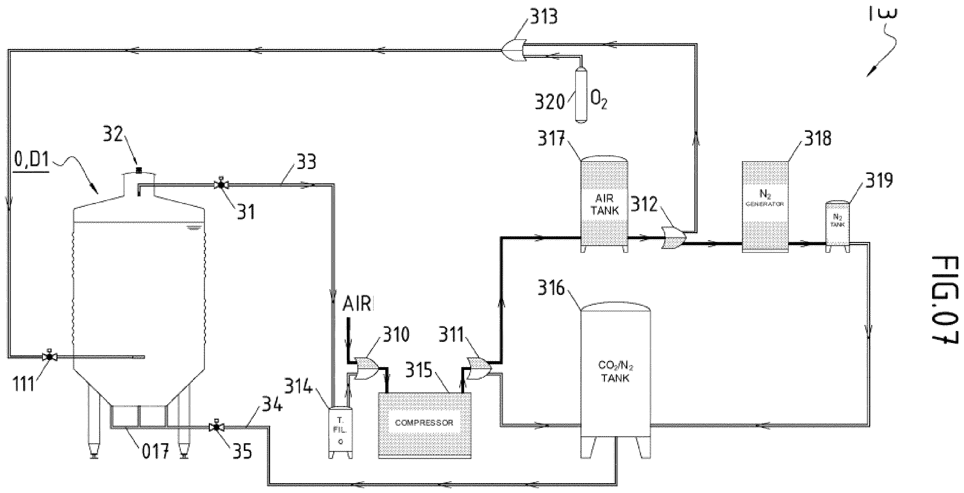

c. a gas processing and storage subsystem for the gases of air, $CO_2$, $N_2$ or $O_2$ (3) (Figures 03-16) which, by means of a plurality of devices and actuators, interacts in the fermentation must, containing in a preferred embodiment an inerting control valve (32), a valve located in the upper part of the tank in the dome element (010) which allows the action of pressurizing the tank (0), incompatible with the vent valve (011), so valve (32) replaces valve (011); and which is supplied from the $CO_2$ tank (0) by means of a suction pipe (33), controlled by an outlet or injection valve (31), a 2-way type seated globe valve having 2 positions that is pneumatically or electrically operated and controlled within the control of the system, the function of which is to allow capturing of $CO_2$ or injecting same or other gases: air or $N_2$; and which, by means of a delivery pipe (34) through which pressurized $CO_2$ or other gases: air or $N_2$, are conducted, controlled by an injection control valve (35), a 2-way type seated globe valve having 2 positions that is pneumatically or electrically operated and controlled within the control of the system, has the function of allowing the entry of $CO_2$ or other gases:

air or $N_2$ to the $CO_2$ injection collector (3, 6), a pipe external to the tank (0) which connects all the $CO_2$ injection elements with the inlet valve (35); and which, by means of an aeration or oxygenation control valve (111), a 2-way type seated globe valve having 2 positions that is pneumatically or electrically operated and controlled within the control of the system, has the function of allowing the $O_2$ or air injection into the tank (0); and which, by means of a cooling control valve (113), a 2-way type seated globe valve having 2 positions that is pneumatically or electrically operated and controlled within the control of the system, has the function of allowing or preventing the passage of liquid coolant to the jackets (05) of the fermentation tank (0).

The subsystem (3) further contains in a preferred embodiment a plurality of valves, these valves being:

- a selector valve for the capturing of $CO_2$ or air (310); being a 3-way type seated globe valve with 3 positions that is pneumatically or electrically operated and controlled within the control subsystem (4); having the function of selecting the capturing of $CO_2$ or air;
- a selector valve for the delivery of $CO_2$ or air (311); being a 3-way type seated globe valve with 3 positions that is pneumatically or electrically operated and controlled within the control subsystem (4); having the function of selecting the delivery of $CO_2$ or air;
- a selector valve for the delivery of air (312); being a 3-way type seated globe valve with 3 positions that is pneumatically or electrically operated and controlled within the control subsystem (4); having the function of selecting the delivery of air;
- a selector valve for the delivery of air or $O_2$ (313); being a 3-way type seated globe valve with 3 positions that is pneumatically or electrically operated and controlled within the control subsystem (4); having the function of selecting the delivery of air or $O_2$.

The subsystem (3) further contains in a preferred embodiment a plurality of tanks, these tanks being:

- trap filter tank (314); this tank prevents the entry of condensate into the compressors, protecting them from said condensate;
- pressurized $CO_2$ or $N_2$ tank (316); being a pressurized buffer tank with a design pressure of 10 bar;
- pressurized air tank (317); being a pressurized buffer tank with a design pressure of 10 bar;
- pressurized $N_2$ tank (319); being a pressurized buffer tank with a design pressure of 10 bar;
- compressed $O_2$ cylinder (320).

The subsystem (3) further contains in a preferred embodiment a compressor (315) with a design pressure of 10 bar according to ISO 8573-1 standard, class 0, in terms of the quality of air suitable for the food industry; and an $N_2$ generator (318); being an $N_2$ generator for inerting the fermentation tank (0).

In a preferred embodiment, the inerting control valve (32) will allow pressurizing in a range of minimum 5 mbar and maximum 40 mbar for large tanks (0) and minimum 5 mbar and maximum 120 mbar for other tanks (0).

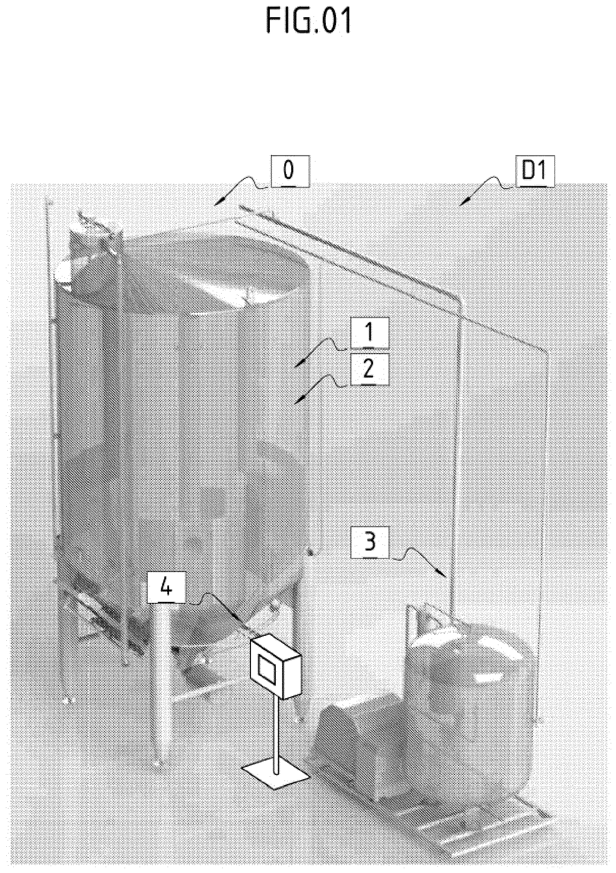

d. a control subsystem (4) (Figure 01) containing in a preferred embodiment:

- a main control panel (40), containing a programmable logic controller (PLC) (401) which incorporates means for communicating with a human machine interface (HMI) device (402);
- a slave panel (41), which incorporates decentralized periphery modules that collect input signals of the instrumentation subsystems for sending data to the panel (40) by means of a single communications bus;
- a secondary panel (42), which incorporates solenoid valve modules that collect control data of the panel (40) by means of a single communications bus in order to act on the valves of the instrumentation subsystems.

*Method for continuous monitoring and improved automatic control of temperature, and of aeration-oxygenation, of the process of alcoholic fermentation in wine by means of acoustic emission techniques (P1) using a system (D1) for the implementation thereof*

A method for continuous monitoring and improved automatic control of temperature of the process of alcoholic fermentation in wine by means of acoustic emission techniques (P1) using a system (D1) for the implementation thereof is described in detail by means of listing the steps to be executed according to the indicated order.

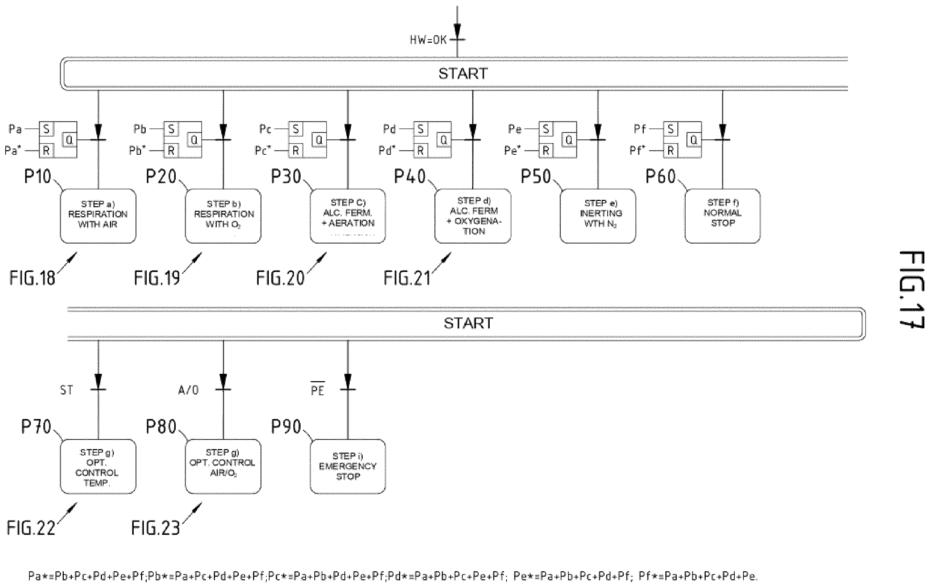

In order to improve understanding and clarity, the operation of each step and sub-step is graphically described by means of Graphs of Control by Steps and Transitions (GRAFCET) and Piping and Instrumentation Diagrams (P&IDs), mentioning in each of them the corresponding figure in each case, avoiding textual description as it is less clear. The P&IDs show the active element in grey and passage through the pipes in black. In the GRAFCET: in selected steps "a-f", the equations of transition of each sub-step, as well as the actuators activated in each of them, are indicated; in steps that are likely to work simultaneously, i.e., steps "g, h, i", the equations of transition of each sub-step, as well as the actuators activated and deactivated in each of them, are indicated. The invention proposes a <u>method</u> for continuous monitoring and improved automatic control of temperature of the process of alcoholic fermentation in wine by means of acoustic emission techniques (PI) using a system (D1) for the implementation thereof, of the type of methods which interact with means for tracking and controlling alcoholic fermentation in a self-emptying fermentation tank (0), comprising a method module implemented in a PLC program (P0), installed in a PLC (401) which incorporates means for communicating with an HMI device (402); wherein if the hardware (HW) of the PLC (401) is correct (OK), a start block is cyclically executed (Figure 17: GRAFCET) which, by means of the interaction of a user in the HMI (402), only calls one of the selected steps "a-f" but with the user being able to activate steps "g, h, i" simultaneously, **characterized in that** the method module implemented in the PLC program (P0) for the purpose of interacting with the means for tracking and controlling comprises at least the following steps:

**STEP "a" (P10): Providing, in a controlled manner, by means of acoustic emission techniques, a fermentation tank (0, D1) with a gas mixture (air + CO₂), allowing yeasts to perform respiration exclusively.**

The passage of air to the fermentation tank (0, D1) is allowed or prevented to perform aeration or oxygenation by means of an aeration or oxygenation control valve (111), and the passage of $CO_2$ to said fermentation tank (0, D1) is allowed or prevented by means of an injection control valve (35).

In this step "a" (P10), it is proposed that the yeast (naturally present or artificially inoculated) mainly performs, by means of the supply of gas (air and $CO_2$), respiration controlled by means of acoustic emission techniques in a fermentation tank (0) in which a device (D1) object of the invention has been implemented.

In this optional step "a" (P10), the object is to reduce the probable alcoholic strength of the wine, causing the yeast to perform respiration by providing it with the required air (of which the yeast will use oxygen), such that a part of the sugars in the must undergoes respiration instead of fermentation. Subsequently, the air supply must be stopped in order to continue with the conventional process of fermentation, since an excess presence of oxygen causes a reduced quality of the wine (González *et al.,* 2013).

The chemical equation (symbolic description) of the chemical reaction of respiration is as follows:

RESPIRATION

$$C_6H_{12}O_6 \text{ (glucose/fructose)} + 6 \cdot O_2 \rightarrow 6 \cdot H_2O \text{ (water)} + 6 \cdot CO_2 + Q \text{ (kJ)}$$

With the following stoichiometry:

- Molecular weight (g/mol):

$$180 + 192 \, (6 \cdot 32) \rightarrow 108 \, (6 \cdot 18) + 264 \, (6 \cdot 44)$$

- Proportion (%):

$$100\% \rightarrow 29.03\% + 70.97\%$$

The $CO_2$ (gas) generated during respiration remains in dissolution until it is released once the fermentation must (liquid) becomes saturated, whereas the water (liquid) remains in dissolution (three times as much $CO_2$ is produced in respiration compared to fermentation).

The $CO_2$ is suctioned for subsequent injection into the fermentation must for the purpose of achieving homogenization, avoiding the performance of pumping over.

During an exhaustive experimentation campaign, it has been found that, in a preferred embodiment, the maximum aeration conditions which maintain the quality of the wine since the level of dissolved oxygen is kept close to zero from the start and the levels of acetic acid at the end of the process are similar to those obtained in an anaerobic process of alcoholic fermentation (0.3 g/l) are:

- *Airflow (l/h) = 5·volume (l) of fermentation must;*
- *Time (h) = 48;*
- *Ethanol reduction = 2% (v/v);*

- *Combined with a discontinuous homogenization cycle by means of $CO_2$ injection.*

Lower aeration conditions may arise maintaining the quality of the wine, but with an ethanol reduction of less than 2% (v/v).

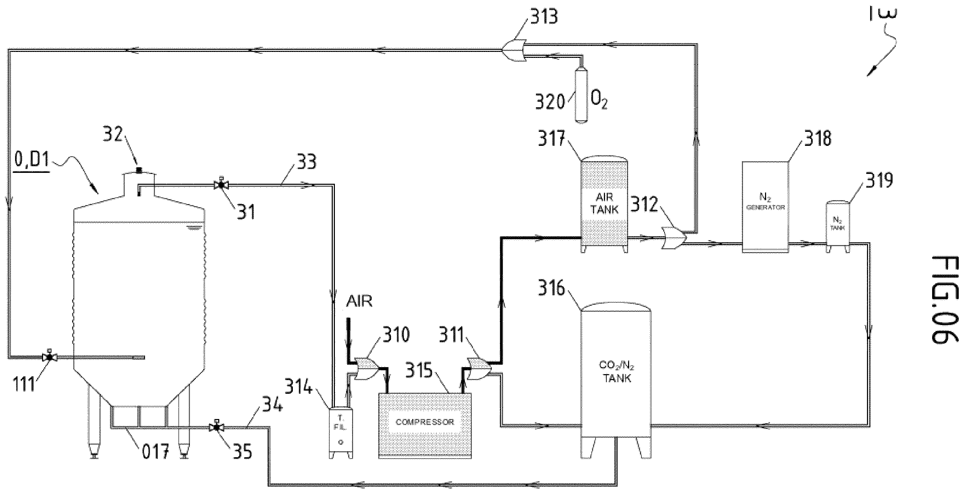

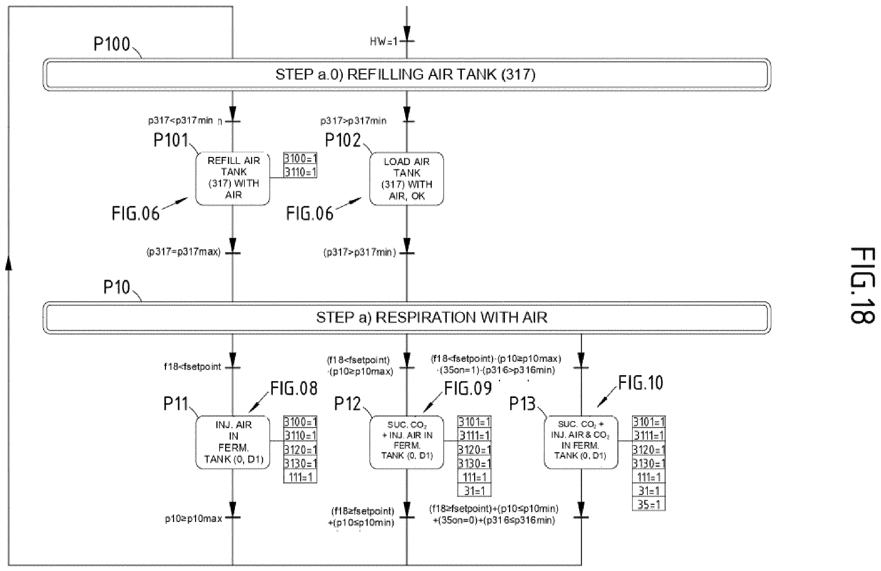

STEP "a" (P10) comprises a prior routine sub-step, STEP "a.0" (P100), of refilling the air tank (317) which is implemented, by way of illustration, in a preferred embodiment of the invention, for the purpose of refilling the air tank (317) within operating limits according to the pressure thereof (Figure 18: GRAFCET; Figure 06: P&ID).

STEP "a" (P10) of respiration with air is characterized in that it comprises at least the following sub-steps (Figure 18: GRAFCET):

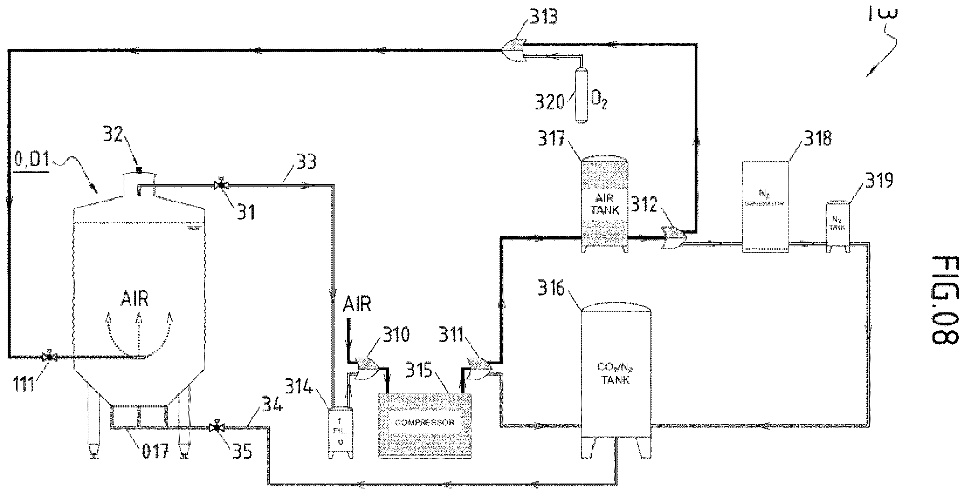

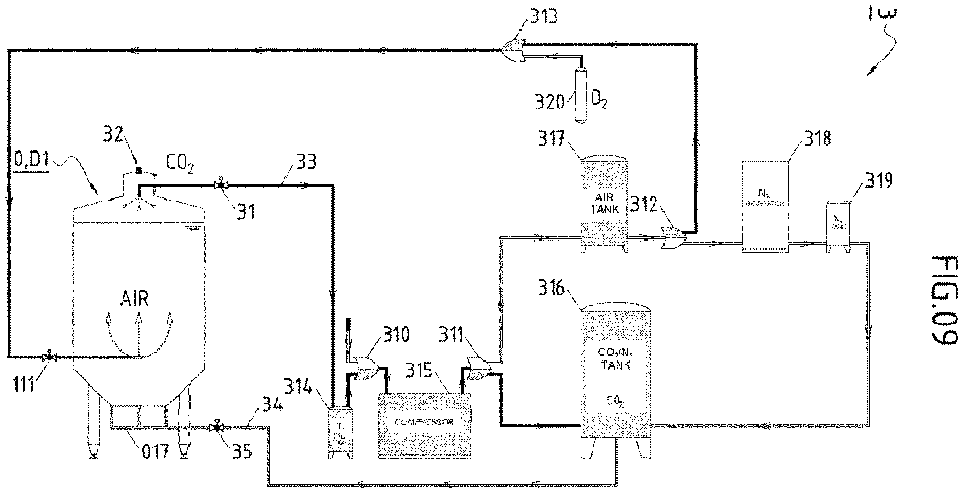

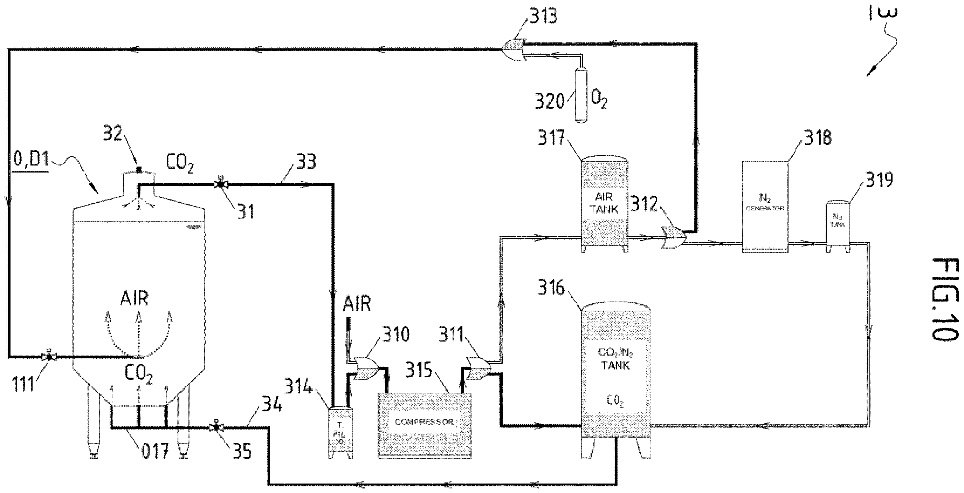

- injecting air in the fermentation tank (0, D1) (P11) (Figure 08: P&ID).
- suctioning $CO_2$ + injecting air in the fermentation tank (0, D1) (P12) (Figure 09: P&ID).
- suctioning $CO_2$ + injecting air and $CO_2$ in the fermentation tank (0, D1) (P13) (Figure 10: P&ID).

The following can be selected simultaneously along with STEP "a" (P10):

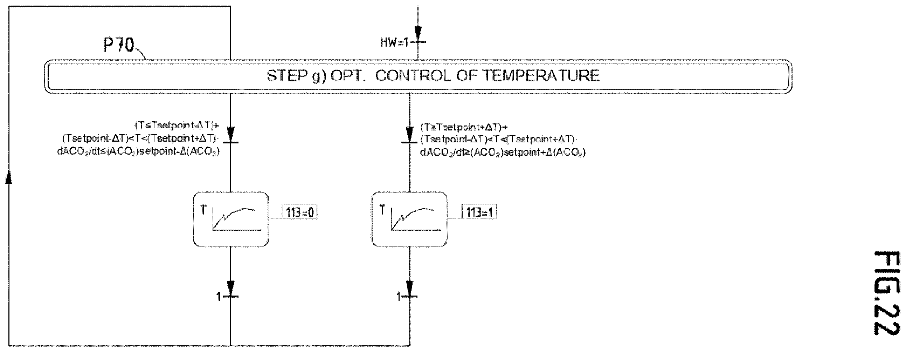

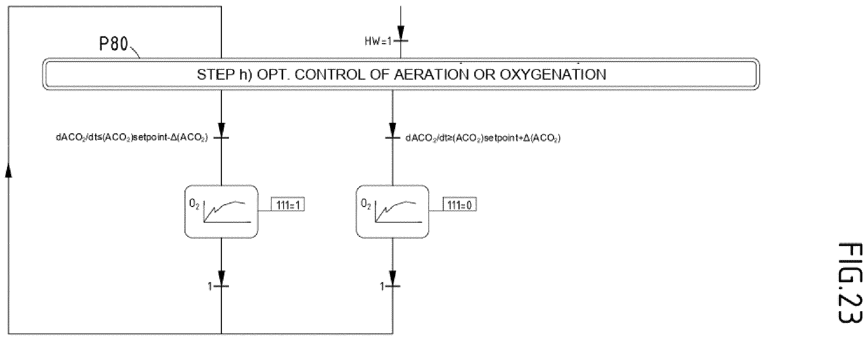

- STEP "g" (P70) of optimized control of temperature in a fermentation tank (0, D1) by means of acoustic emission techniques (Figure 22: GRAFCET).
- STEP "h" (P80) of optimized control of aeration or oxygenation in a fermentation tank (0, D1) by means of acoustic emission techniques (Figure 23: GRAFCET).

**STEP "b" (P20): Providing, in a controlled manner, by means of acoustic emission techniques, a fermentation tank (0, D1) with a gas mixture ($O_2$ + $CO_2$), allowing yeasts to perform respiration exclusively.**

The passage of $O_2$ to the fermentation tank (0, D1) is allowed or prevented to perform aeration or oxygenation by means of an aeration or oxygenation control valve (111), and the passage of $CO_2$ to said fermentation tank (0, D1) is allowed or prevented by means of an injection control valve (35).

In this STEP "b" (P20), it is proposed that the yeast (naturally present or artificially inoculated) mainly performs, by means of the supply of gas ($O_2$ and $CO_2$), respiration controlled by means of acoustic emission techniques in a fermentation tank (0) in which a device (D1) object of the invention has been implemented.

STEP "b" is similar to STEP "a" but with the difference that a gas mixture containing $O_2$ instead of air is provided.

During an exhaustive experimentation campaign, it has been found that, in a preferred embodiment, the maximum oxygenation conditions which maintain the quality of the wine since the level of dissolved oxygen is kept close to zero from the start and the levels of acetic acid at the end of the process are similar to those obtained in an anaerobic process of alcoholic fermentation (0.3 g/l) are:

- *Oxygen flow (l/h) = 1·volume (l) of fermentation must;*
- *Time (h) = 48;*
- *Ethanol reduction = 2% (v/v);*
- *Combined with a discontinuous homogenization cycle by means of $CO_2$ injection.*

Lower oxygenation conditions can arise maintaining the quality of the wine, but with an ethanol reduction of less than 2% (v/v).

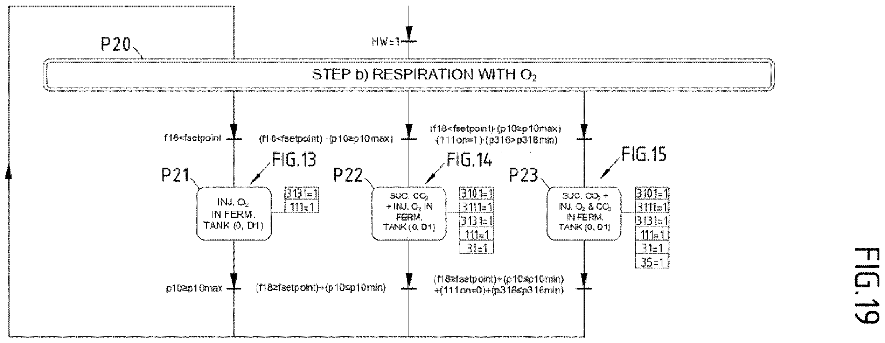

STEP "b" (P20) of respiration with $O_2$ is characterized in that it comprises at least the following sub-steps (Figure 19: GRAFCET):

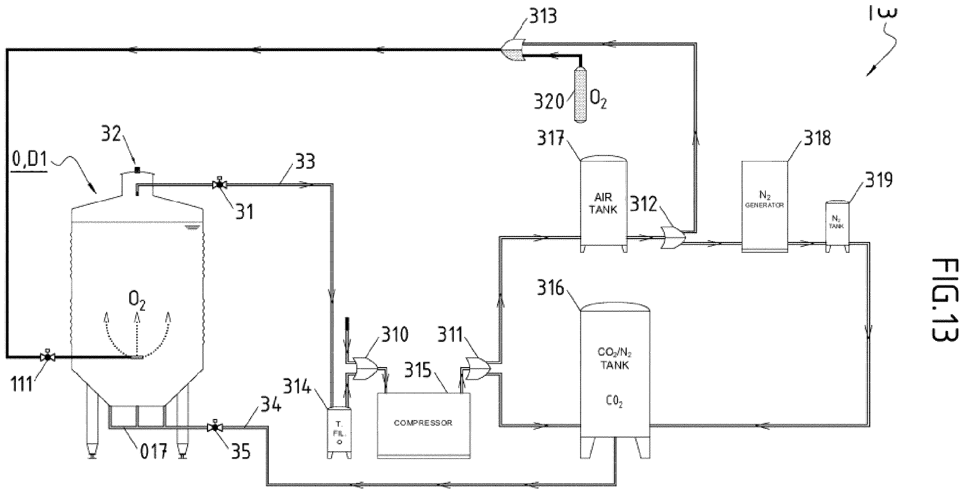

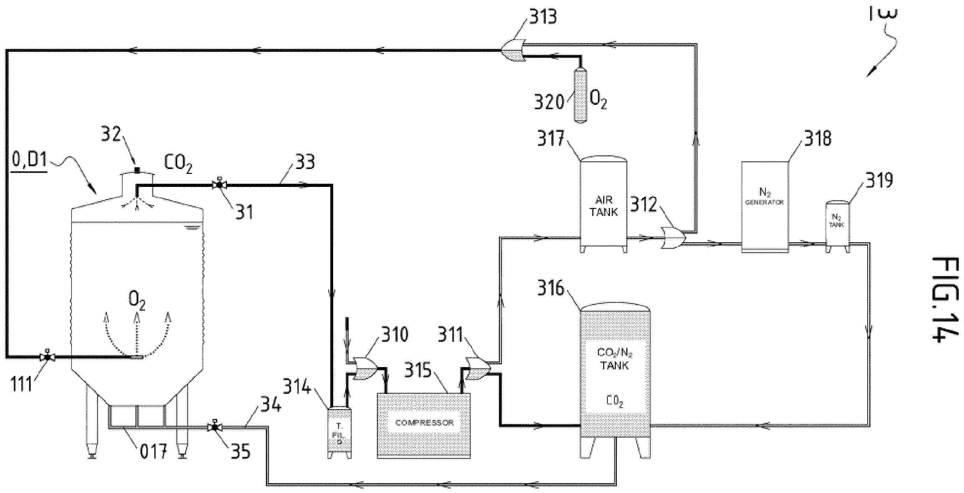

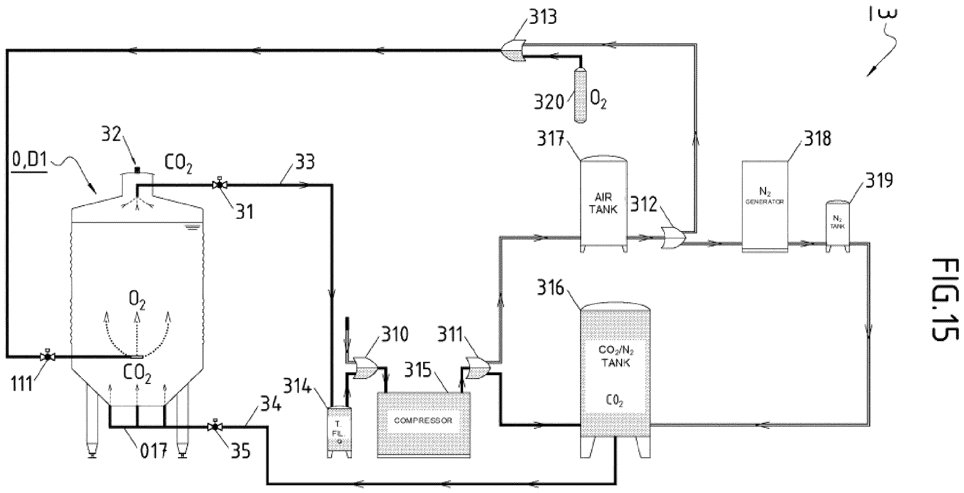

- injecting $O_2$ in the fermentation tank (0, D1) (P21) (Figure 13: P&ID).
- suctioning $CO_2$ + injecting $O_2$ in the fermentation tank (0, D1) (P22) (Figure 14: P&ID).
- suctioning $CO_2$ + injecting $O_2$ and $CO_2$ in the fermentation tank (0, D1) (P23) (Figure 15: P&ID).

The following can be selected simultaneously along with STEP "b" (P20):

- STEP "g" (P70) of optimized control of temperature in a fermentation tank (0, D1) by means of acoustic emission techniques (Figure 22: GRAFCET).
- STEP "h" (P80) of optimized control of aeration or oxygenation in a fermentation tank (0, D1) by means of acoustic emission techniques (Figure 23: GRAFCET).

**STEP "c" (P30): Providing a fermentation tank (0, D1) with a gas mixture (CO$_2$ + air), allowing yeasts to perform combined alcoholic fermentation and respiration monitored and controlled by means of acoustic emission techniques.**

The passage of air to the fermentation tank (0, D1) is allowed or prevented to perform aeration or oxygenation by means of an aeration or oxygenation control valve (111), and the passage of CO$_2$ to said fermentation tank (0, D1) is allowed or prevented by means of an injection control valve (35).

In this STEP "c" (P30), it is proposed that the yeast (naturally present or artificially inoculated) mainly performs, by means of the supply of gas (CO$_2$ and air), alcoholic fermentation monitored and controlled by means of acoustic emission techniques in a fermentation tank (0) in which a device (D1) object of the invention has been implemented.

The chemical equation (symbolic description) of the chemical reaction of alcoholic fermentation is as follows:

FERMENTATION

$$C_6H_{12}O_6 \text{ (glucose/fructose)} \rightarrow 2 \cdot C_2H_5OH \text{ (ethanol)} + 2 \cdot CO_2 + Q \text{ (kJ)}$$

With the following stoichiometry:

- Molecular weight (g/mol); energy (kcal/mol of sugar consumed):

$$180 \rightarrow 92 \ (2 \cdot 46) + 88 \ (2 \cdot 44) + 23.5 \text{ kcal}$$

- Proportion (%):

$$100\% \rightarrow 51.11\% + 48.89\%$$

The CO$_2$ (gas) generated during alcoholic fermentation remains in dissolution until it is released once the fermentation must (liquid) becomes saturated, whereas ethanol (liquid) remains in dissolution, although a small fraction of ethanol is lost through evaporation.

The CO$_2$ is suctioned for subsequent injection into the fermentation must for the purpose of achieving homogenization, avoiding the performance of pumping over.

The aeration in this STEP "c" allows the yeast to also perform respiration for the purpose of facilitating its growth and reproduction by providing it with the required air (of which the yeast will use oxygen), such that a part of the sugars in the must undergoes respiration instead of fermentation. STEP "c" differs from STEP "a" in that in STEP "a" respiration is performed continuously for some time, whereas in this STEP "c" respiration can be performed discontinuously or continuously together with fermentation, producing wines with more color that is fixed by the oxygen from aeration, in addition to achieving a greater disassociation of tannins and anthocyanins.

The maximum aeration conditions which maintain the quality of the wine since the level of dissolved oxygen is kept close to zero from the start and the levels of acetic acid at the end of the process are similar to those obtained in an anaerobic process of alcoholic fermentation (0.3 g/l) are the same as those found during the experimentation campaign described in STEP "a", these conditions being:

- *Airflow (l/h) = 5·volume (l) of fermentation must;*
- *Time (h) = 48 (counted in a continuous regimen or accumulated in a discontinuous regimen);*
- *Ethanol reduction = 2% (v/v);*
- *Combined with a discontinuous homogenization cycle by means* of CO$_2$ injection.

Lower aeration conditions can arise maintaining the quality of the wine, but with an ethanol reduction of less than 2% (v/v).

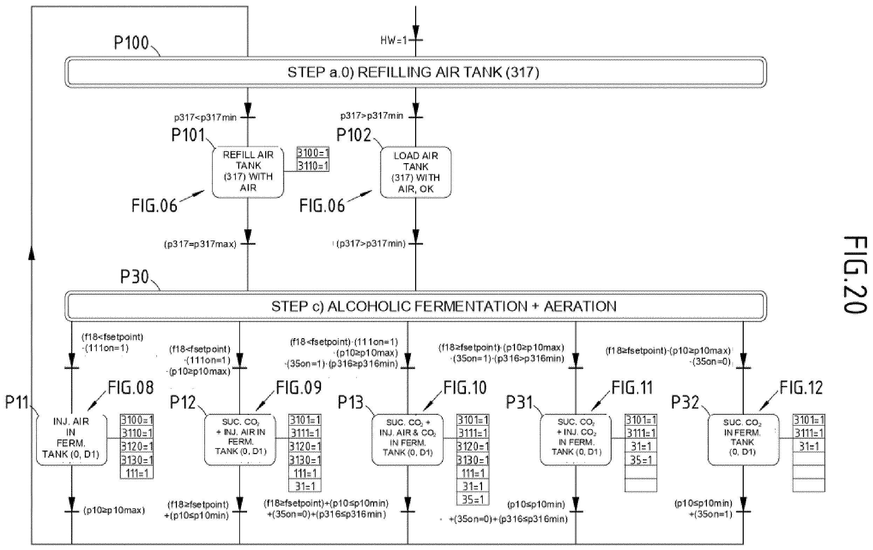

This step comprises a prior routine sub-step, STEP "a.0" (P100), of refilling the air tank (317) which is implemented, by way of illustration, in a preferred embodiment of the invention, for the purpose of refilling the air tank (317) within operating limits according to the pressure thereof (Figure 20: GRAFCET; Figure 06: P&ID).

STEP "c" (P30) of alcoholic fermentation plus aeration is characterized in that it comprises at least the following sub-steps (Figure 20: GRAFCET):

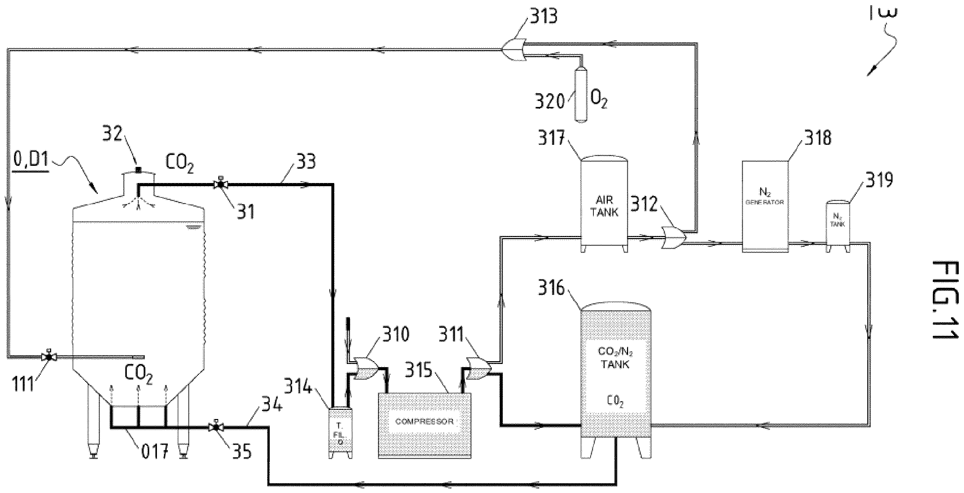

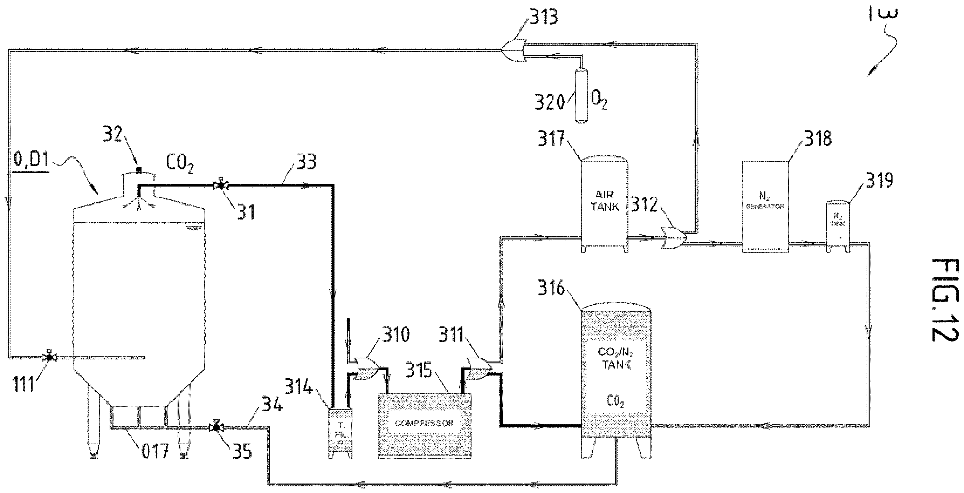

- injecting air in the fermentation tank (0, D1) (P11) (Figure 08: P&ID).
- suctioning $CO_2$ + injecting air in the fermentation tank (0, D1) (P12) (Figure 09: P&ID).
- suctioning $CO_2$ + injecting air and $CO_2$ in the fermentation tank (0, D1) (P13) (Figure 10: P&ID).
- suctioning $CO_2$ + injecting $CO_2$ in the fermentation tank (0, D1) (P31) (Figure 11: P&ID).
- suctioning $CO_2$ in the fermentation tank (0, D1) (P32) (Figure 12: P&ID).

The following can be selected simultaneously along with STEP "c" (P30):

- STEP "g" (P70) of optimized control of temperature in a fermentation tank (0, D1) by means of acoustic emission techniques (Figure 22: GRAFCET).
- STEP "h" (P80) of optimized control of aeration or oxygenation in a fermentation tank (0, D1) by means of acoustic emission techniques (Figure 23: GRAFCET).

**STEP "d" (P40): Providing a fermentation tank (0, D1) with a gas mixture ($CO_2$ + $O_2$), allowing yeasts to perform combined alcoholic fermentation and respiration monitored and controlled by means of acoustic emission techniques.**

The passage of $O_2$ to the fermentation tank (0, D1) is allowed or prevented to perform aeration or oxygenation by means of an aeration or oxygenation control valve (111), and the passage of $CO_2$ to said fermentation tank (0, D1) is allowed or prevented by means of an injection control valve (35).
In this STEP "d" (P40), it is proposed that the yeast (naturally present or artificially inoculated) mainly performs, by means of the supply of gas ($CO_2$ and $O_2$), alcoholic fermentation monitored and controlled by means of acoustic emission techniques in a fermentation tank (0) in which a device (D1) object of the invention has been implemented.
STEP "d" is similar to STEP "c" but with the difference that a gas mixture containing $O_2$ instead of air is provided.
The oxygenation of this STEP "d" allows the yeast to also perform respiration for the purpose of facilitating its growth and reproduction by providing it with the required oxygen, such that a part of the sugars in the must undergoes respiration instead of fermentation. STEP "d" differs from STEP "b" in that in STEP "b" respiration is performed continuously for some time, whereas in this STEP "d" respiration can be performed discontinuously or continuously together with fermentation, producing, like in the preceding step, wines with more color that is fixed by the oxygen from oxygenation, in addition to achieving a greater disassociation of tannins and anthocyanins.
The maximum oxygenation conditions which maintain the quality of the wine since the level of dissolved oxygen is kept close to zero from the start and the levels of acetic acid at the end of the process are similar to those obtained in an anaerobic process of alcoholic fermentation (0.3 g/l) are the same as those found during the experimentation campaign described in STEP "b", these conditions being:

- *Oxygen flow (l/h) = 1· V (l) of fermentation must;*
- *Time (h) = 48 (counted in a continuous regimen or accumulated in a discontinuous regimen);*
- *Ethanol reduction = 2% (v/v);*
- *Combined with a discontinuous homogenization cycle by means* of $CO_2$ *injection.*

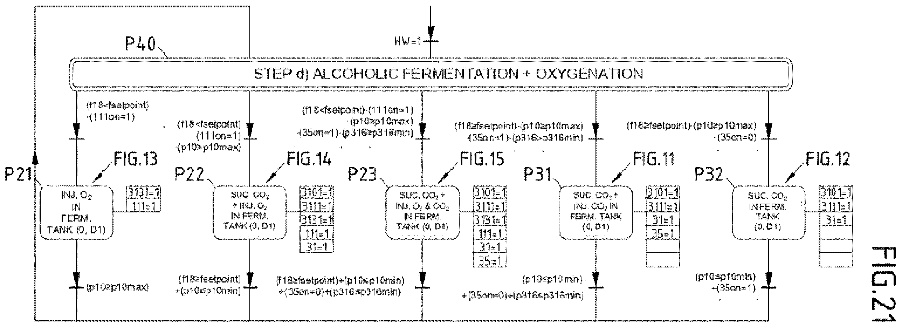

Lower oxygenation conditions may arise maintaining the quality of the wine, but with an ethanol reduction of less than 2% (v/v).
STEP "d" (P40) of alcoholic fermentation plus oxygenation is characterized in that it comprises at least the following sub-steps (Figure 21: GRAFCET):

- injecting $O_2$ in the fermentation tank (0, D1) (P13) (Figure 13: P&ID).
- suctioning $CO_2$ + injecting $O_2$ in the fermentation tank (0, D1) (P22) (Figure 14: P&ID).
- suctioning $CO_2$ + injecting $O_2$ and $CO_2$ in the fermentation tank (0, D1) (P23) (Figure 15: P&ID).
- suctioning $CO_2$ + injecting of $CO_2$ in the fermentation tank (0, D1) (P31) (Figure 11: P&ID).
- suctioning $CO_2$ in the fermentation tank (0, D1) (P32) (Figure 12: P&ID).

The following can be selected simultaneously along with STEP "d" (P40):

- STEP "g" (P70) of optimized control of temperature in a fermentation tank (0, D1) by means of acoustic emission techniques (Figure 22: GRAFCET).
- STEP "h" (P80) of optimized control of aeration or oxygenation in a fermentation tank (0, D1) by means of acoustic emission techniques (Figure 23: GRAFCET).

**STEP "e" (P50): Providing a fermentation tank (0, D1) with gas (N$_2$) for the purpose of inerting the tank.**

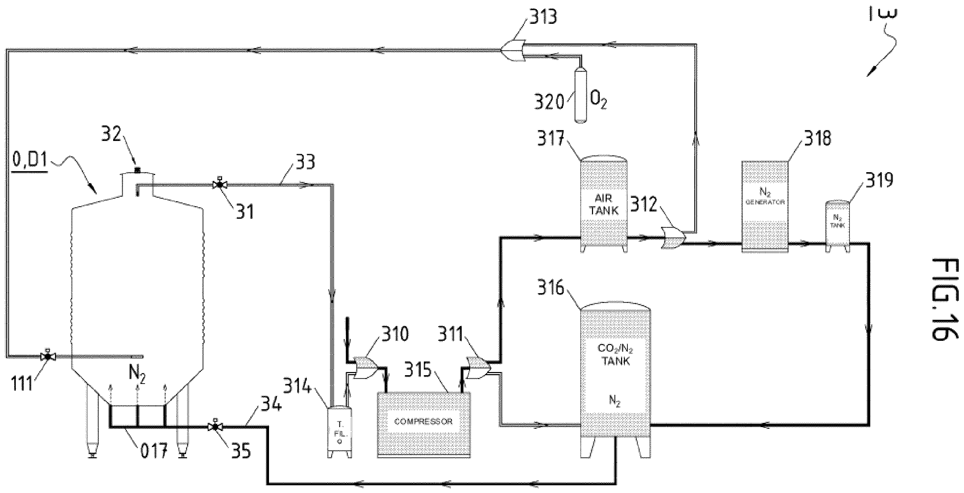

A routine step which is implemented, by way of illustration, in a preferred embodiment of the invention (Figure 21: GRAFCET) (Figure 16: P&ID).

STEP "f" (P60): Performing a normal stop in the control of a fermentation tank (0, **D1).**

A routine step which is implemented, by way of illustration, in a preferred embodiment of the invention (Figure 21: GRAFCET). By selecting STEP "f" (P60) any of steps "a-f" that was selected is deselected, so the actuations of steps "a-f" transition to the logic state = 0. In a preferred embodiment, simultaneous STEPS "g-h" are kept in operation.

**STEP "g" (P70): Monitoring and controlling the temperature in a fermentation tank (0, D1) by means of acoustic emission techniques.**

Since alcoholic fermentation is an exothermic reaction, the heat generated during the process of fermentation must be removed in order to maintain the optimal temperature. To that end, a cooling system of any of the state of the art is used which, by means of a cooling control valve (113), allows or prevents the passage of the liquid coolant to the jackets (05) of the fermentation tank (0, D1).

One of the parameters having the most influence on the evolution of the process of alcoholic fermentation is temperature which, in addition to an optimum temperature, contributes to obtaining wine with the typical characteristics of its denomination. In general, wines prepared at low temperature are more aromatic and better valued in the organoleptic analysis but, on the other hand, their fermentation can be incomplete.

The acoustic emission ($ACO_2$) caused by the $CO_2$ generated during respiration and alcoholic fermentation is measured and the acoustic emission speed ($dACO_2/dt$) is calculated for the early detection of the fermentation speed (g of $CO_2$/l/h) (amount, by weight or by volume, of $CO_2$ produced per unit of time), and therefore the activity of the yeasts.

In a preferred embodiment, the analog signal of the omnidirectional hydrophone sensor (20) is collected in the slave panel (41), being sent to the main control panel (40) and reaching the programmable logic controller (401) where it is internally converted to a digitalized signal, first being processed in a calibration module (P101) of the program (P0) to obtain the basic amplitude, frequency, and spectral descriptors thereof by applying fast Fourier transform (FFT); with the correct frequency range, the digitalized signal passes to the filtration module (P102) where a digital filter is applied to the signal to discriminate frequencies not related with the process of fermentation, finally obtaining the real-time value of the acoustic emission ($ACO_2$).

According to another configuration for obtaining acoustic emission ($ACO_2$), noise from aeration, oxygenation, $CO_2$ injection recirculation of cooling water through the jackets, and any other desired noise is eliminated; to that end, before the start of alcoholic fermentation the acoustic emission of aeration (AAera), of oxygenation ($AO_2$), of $CO_2$ injection ($AICO_2$), of cooling (AR), and of any other desired noise (AO) is recorded, then, noise is eliminated from the acoustic emission measured in the fermentation must (AMF) by means of the following equation:

$$ACO_2 = AMF - AAera - AO_2 - AICO_2 - AR - AO$$

since the PLC program (PC), installed in the PLC (401), has at all times the state of the aeration, oxygenation, $CO_2$ injection, and cooling sub-steps so that in the case where one or more of them are activated, the corresponding noise from the active sub-steps can be subtracted by means of the preceding equation; that which is indicated above is performed if a noise emitting source is detected for the purpose of obtaining only the acoustic emission ($ACO_2$) caused by the $CO_2$ generated during respiration and alcoholic fermentation.

When the acoustic emission speed ($dACO_2/dt$), i.e., $CO_2$ production, exceeds a previously established limit (($ACO_2$)setpoint), taking into account a preestablished hysteresis value ($\Delta(ACO_2)$), the cooling control valve (113) is opened to keep the temperature within compatible winemaking limits to stabilize alcoholic fermentation, as shown in the following simple conditional logic equation (Figure 22: GRAFCET):

$$\text{"If" } 113 = 0 \text{ "AND" } T \geq (T_{setpoint} + \Delta T) \text{ "O"}$$

$$(T_{setpoint} - \Delta T) < T < (T_{setpoint} + \Delta T) \text{ "AND" } \left( \frac{dACO_2}{dt} \geq ((ACO_2)setpoint + \Delta(ACO_2)) \right)$$

$$\text{"then" } \{113 = 1\}$$

When the acoustic emission speed ($dACO_2/dt$), i.e. production of $CO_2$, is below a previously established limit (($ACO_2$)setpoint), taking into account a preestablished hysteresis value ($\Delta(ACO_2)$), the cooling control valve (113) is closed allowing the temperature to rise to within compatible winemaking limits in order to reactivate alcoholic fermentation, as shown in the following simple conditional logic equation (Figure 22: GRAFCET):

$$\text{"If" } 113 = 1 \text{ "AND" } T \leq (\text{Tsetpoint} - \Delta T) \text{ "O"}$$

$$(\text{Tsetpoint} - \Delta T) < T < (\text{Tsetpoint} + \Delta T) \text{"AND"} \left( \frac{dACO_2}{dt} \leq ((ACO_2)\text{setpoint} - \Delta(ACO_2)) \right)$$

$$\text{"then" } \{113 = 0\}$$

This set of indicated operations allows an optimum alcoholic fermentation kinetics (speed), as well as a quicker completion of fermentation.

### STEP "h" (P80): Monitoring and controlling the aeration or oxygenation of a fermentation tank (0, D1) by means of acoustic emission techniques.

The passage of air or oxygen to the fermentation tank (0, D1) is allowed or prevented to perform aeration or oxygenation by means of an aeration or oxygenation control valve (111).

The acoustic emission ($ACO_2$) caused by the $CO_2$ generated during respiration and alcoholic fermentation is measured and the acoustic emission speed ($dACO_2/dt$) is calculated for the early detection of the fermentation speed (g of $CO_2$/l/h), and therefore the activity of the yeasts.

In a preferred embodiment, the analog signal of the omnidirectional hydrophone sensor (20) is collected in the slave panel (41), being sent to the main control panel (40) and reaching the programmable logic controller (401) where it is internally converted to a digitalized signal, first being processed in a calibration module (P101) of the program (P0) to obtain the basic amplitude, frequency, and spectral descriptors thereof by applying fast Fourier transform (FFT); with the correct frequency range, the digitalized signal passes to the filtration module (P102) where a digital filter is applied to the signal to discriminate frequencies not related with the process of fermentation, finally obtaining the real-time value of the acoustic emission ($ACO_2$).

According to another configuration for obtaining acoustic emission ($ACO_2$), noise from aeration, oxygenation, $CO_2$ injection recirculation of cooling water through the jackets, and of any other desired noise is eliminated; to that end, before the start of alcoholic fermentation the acoustic emission of aeration (AAera), of oxygenation (AO$_2$), of $CO_2$ injection (AICO$_2$), of cooling (AR), and any other desired noise (AO) is recorded, then, noise is eliminated from the acoustic emission measured in the fermentation must (AMF) by means of the following equation:

$$ACO_2 = AMF - AAera - AO_2 - AICO_2 - AR - AO$$

since the PLC program (PC), installed in the PLC (401), has at all times the state of the aeration, oxygenation, $CO_2$ injection, and cooling sub-steps so that in the case where one or more of them are activated, the corresponding noise from the active sub-steps can be subtracted by means of the preceding equation; that which is indicated above is performed if a noise emitting source is detected for the purpose of obtaining only the acoustic emission ($ACO_2$) caused by the $CO_2$ generated during respiration and alcoholic fermentation.

When the acoustic emission speed ($dACO_2/dt$), i.e., $CO_2$ production, is below a previously established limit (($ACO_2$)setpoint), taking into account a preestablished hysteresis value ($\Delta(ACO_2)$), the aeration or oxygenation control valve (111) is opened in order to reactivate the activity of the yeasts, as shown in the following simple conditional logic equation (Figure 23: GRAFCET):

$$\text{"If" } 111 = 0 \text{"AND"} \left( \frac{dACO_2}{dt} \leq ((ACO_2)\text{setpoint} - \Delta(ACO_2)) \right)$$

$$\text{"then" } \{111 = 1\}$$

When the acoustic emission speed ($dACO_2/dt$), i.e., $CO_2$ production, exceeds a previously established limit (($ACO_2$)setpoint), taking into account a preestablished hysteresis value ($\Delta(ACO_2)$), the aeration or oxygenation control valve (111) is closed in order to deactivate the excessive activity of the yeasts, as shown in the following simple conditional logic equation (Figure 23: GRAFCET):

$$\text{"If" } 111 = 1 \text{"AND"} \left( \frac{dACO_2}{dt} \geq ((ACO_2)\text{setpoint} + \Delta(ACO_2)) \right)$$

$$\text{"then" } \{111 = 0\}$$

This operation allows aeration or oxygenation metering to be optimized, as required, until the accumulated flow (f18), measured by the aeration or oxygenation flowmeter sensor (18), reaches the setpoint value (fsetpoint), obtaining an increase in wine quality in the Total Polyphenol Index (TPI) which causes a qualitative leap in terms of organoleptic parameters.

STEP "i" (P90): <u>Performing an emergency stop in the control of a fermentation tank (0, D1).</u>

A routine step which is implemented, by way of illustration, in a preferred embodiment of the invention (Figure 21: GRAFCET). By actuating the NC emergency push button (PE), STEP "i" (P90) which will reset all the actuations is selected.

**Claims**

1. Device for continuous monitoring and improved automatic control of temperature, and of aeration-oxygenation, of the process of alcoholic fermentation in wine by means of acoustic emission techniques (D1), of the type of devices that incorporate means for tracking and controlling alcoholic fermentation in a self-emptying fermentation tank (0), and that is **characterized in that** the means for tracking and controlling comprise:

    a. an instrumentation subsystem (1) which, by means of a plurality of sensors, measures and calculates a series of parameters in the fermentation must, containing:

    A set of sensors arranged in the self-emptying fermentation tank (0) in the torispherical head, these sensors being:

       - a torispherical head pressure sensor (10) that measures the pressure (P) of $CO_2$ or other gases: air or $N_2$, and it is combined with sensors (13, 15) to eliminate calculation error of the density (D) of the fermentation must;
       - a torispherical head temperature sensor (11) that measures the temperature (T) of $CO_2$ or other gases: air or $N_2$;
       - a torispherical head flowmeter sensor (12) that measures the flow rate (F) of suctioned $CO_2$ or of other gases: injected air or $N_2$.

    A set of sensors arranged in the self-emptying fermentation tank (0) in the upper cylindrical body, these sensors being:

       - an upper cylindrical body pressure sensor (13) that measures the pressure (P) in the liquid phase of the fermentation must below the area of the "cap" combined with the sensor (15) and, by eliminating error by means of the sensor (10), the density (D) of the must and the level of the free surface are calculated;
       - an upper cylindrical body temperature sensor (14) that measures the temperature (T) in the fermentation must below the area of the "cap".

    A set of sensors arranged in the self-emptying fermentation tank (0) in the lower cylindrical body, these sensors being:

       - a lower cylindrical body pressure sensor (15) that measures the pressure (P) in the liquid phase of the fermentation must above the area of solids sunken to the bottom combined with the sensor (13) and, by eliminating error by means of the sensor (10), the density (D) of the must and the level of the free surface are calculated;
- a lower cylindrical body temperature sensor (16) that measures the temperature (T) in the liquid phase of the fermentation must above the area of solids sunken to the bottom;
- an aeration or oxygenation flowmeter sensor (18) that measures the flow rate (F) of injected air or $O_2$.

A $CO_2$ or $N_2$ injection flowmeter sensor (17) that measures the flow rate (F) of injected $CO_2$ or $N_2$, arranged in the self-emptying fermentation tank (0) in the toriconical end.

b. an acoustic emission instrumentation subsystem (2) that measures the acoustic emission caused by the $CO_2$ generated during respiration and alcoholic fermentation in the fermentation must, containing:

- an omnidirectional-type hydrophone sensor (20) that measures the acoustic emission (A) produced within the fermentation must;
- an analog signal conditioner (21) that pre-amplifies (AS) the voltage of the hydrophone (20).

c. a gas processing and storage subsystem for the gases of air, $CO_2$ $N_2$ or $O_2$ (3) which, by means of a plurality of devices and actuators, interacts in the fermentation must, containing in a preferred embodiment an inerting control valve (32), a valve located in the upper part of the tank in the dome element (010) which allows the action of pressurizing the tank (0), incompatible with the vent valve (011), so valve (32) replaces valve (011); and which is supplied from the $CO_2$ tank (0) by means of a suction pipe (33), controlled by an outlet or injection valve (31), a 2-way type seated globe valve having 2 positions that is pneumatically or electrically operated and controlled within the control of the system, the function of which is to allow capturing of $CO_2$ or injecting same or other gases: air or $N_2$; and which, by means of a delivery pipe (34) through which pressurized $CO_2$ or other gases: air or $N_2$, are conducted, controlled by an injection control valve (35), a 2-way type seated globe valve having 2 positions that is pneumatically or electrically operated and controlled within the control of the system, has the function of allowing the entry of $CO_2$ or other gases: air or $N_2$ to the $CO_2$ injection collector (36), a pipe external to the tank (0) which connects all the $CO_2$ injection elements with the inlet valve (35); and which, by means of an aeration or oxygenation control valve (111), a 2-way type seated globe valve having 2 positions that is pneumatically or electrically operated and controlled within the control of the system, has the function of allowing $CO_2$ or air injection into the tank (0); and which, by means of a cooling control valve (113), a 2-way type seated globe valve having 2 positions that is pneumatically or electrically operated and controlled within the control of the system, has the function of allowing or preventing the passage of liquid coolant to the jackets (05) of the fermentation tank (0).

The subsystem (3) further contains a plurality of valves, these valves being:

- a selector valve for the capturing of $CO_2$ or air (310), a 3-way type seated globe valve with 3 positions that is pneumatically or electrically operated and controlled within the control subsystem (4), and having the function of selecting the capturing of $CO_2$ or air;
- a selector valve for the delivery of $CO_2$ or air (311), a 3-way type seated globe valve with 3 positions that is pneumatically or electrically operated and controlled within the control subsystem (4), and having the function of selecting the delivery of $CO_2$ or air;
- a selector valve for the delivery of air (312), a 3-way type seated globe valve with 3 positions that is pneumatically or electrically operated and controlled within the control subsystem (4), and having the function of selecting the delivery of air;
- a selector valve for the delivery of air or $O_2$ (313), a 3-way type seated globe valve with 3 positions that is pneumatically or electrically operated and controlled within the control subsystem (4), and having the function of selecting the delivery of air or $O_2$;

The subsystem (3) further contains a plurality of tanks, these tanks being:

- trap filter tank (314) which prevents the entry of condensate into the compressors, protecting them from said condensate;
- pressurized $CO_2$ or $N_2$ tank (316), which is a pressurized buffer tank with a design pressure of 10 bar;
- pressurized air tank (317), which is a pressurized buffer tank with a design pressure of 10 bar;
- pressurized $N_2$ tank (319), which is a pressurized buffer tank with a design pressure of 10 bar;
- compressed $O_2$ cylinder (320).

The subsystem (3) further comprises a compressor (315) with a design pressure of 10 bar, suitable for the food industry, and an $N_2$ generator (318) which is an $N_2$ generator for inerting the fermentation tank (0).

   d. a control subsystem (4), containing:

      - a main control panel (40), containing a programmable logic controller (PLC) (401) which incorporates means for communicating with a human machine interface (HMI) device (402);
      - a slave panel (41), which incorporates decentralized periphery modules that collect input signals of the instrumentation subsystems for sending data to the panel (40) by means of a single communications bus;
      - a secondary panel (42), which incorporates solenoid valve modules that collect control data of the panel (40) by means of a single communications bus in order to act on the valves of the instrumentation subsystems.

2. Device according to claim 1, **characterized by** the fact that the omnidirectional-type hydrophone sensor (20) has a range of 1 Hz to 140 kHZ, of horizontal positioning and $\pm 2$dB omnidirectional type with an operating temperature range of -2°C to +80°C, of the type that is submersible and resistant to wine must and acetic acid, and is arranged submerged in the fermentation must contained in the self-emptying fermentation tank (0).

3. Method for continuous monitoring and improved automatic control of temperature, and of aeration-oxygenation, of the process of alcoholic fermentation in wine by means of acoustic emission techniques (P1) by means of using a system (D1) for the implementation thereof, of the type of methods which interact with means for tracking and controlling alcoholic fermentation in a self-emptying fermentation tank (0), comprising a method module implemented in a PLC program (P0), installed in a PLC (401) which incorporates means for communicating with an HMI device (402), wherein if the hardware (HW) of the PLC (401) is correct (OK), a start block is cyclically executed which, by means of the interaction of a user in the HMI (402), only calls one of the selected steps "a-f" but with the user being able to activate steps "g, h, i" simultaneously, **characterized in that** the method module implemented in the PLC program (PC) for the purpose of interacting with the means for tracking and controlling comprises at least the following steps:

   - **STEP "a" (P10): Providing, in a controlled manner, by means of acoustic emission techniques, a fermentation tank (0, D1) with a gas mixture (air + $CO_2$), allowing yeasts to perform respiration exclusively.**
   The passage of air to the fermentation tank (0, D1) is allowed or prevented to perform aeration by means of an aeration or oxygenation control valve (111), and the passage of $CO_2$ to said fermentation tank (0, D1) is allowed or prevented by means of an injection control valve (35). In this step "a" (P10), the naturally present or artificially inoculated yeast performs, by means of the supply of gas (air and $CO_2$), respiration controlled by means of acoustic emission techniques in a fermentation tank (0) in which a device (D1) object of the invention has been implemented. In this optional step "a" (P10), the probable alcoholic strength of the wine is reduced, causing the yeast to perform respiration by providing it with the required air (of which the yeast will use oxygen), such that a part of the sugars in the must undergoes respiration instead of fermentation. Subsequently, the air supply must be stopped in order to continue with the conventional process of fermentation, since an excess presence of oxygen causes a reduced quality of the wine. The $CO_2$ (gas) generated during respiration remains in dissolution until it is released once the fermentation must (liquid) becomes saturated, whereas the water (liquid) remains in dissolution (three times as much $CO_2$ is produced in respiration compared to fermentation). The $CO_2$ is suctioned for subsequent injection into the fermentation must for the purpose of achieving homogenization, avoiding the performance of pumping over.
   STEP "a" (P10) of respiration with air comprises at least the following sub-steps:

      - injecting air in the fermentation tank (0, D1) (P11);
      - suctioning $CO_2$ + injecting air in the fermentation tank (0, D1) (P12);
      - suctioning $CO_2$ + injecting air and $CO_2$ in the fermentation tank (0, D1) (P13).

   The following can be selected simultaneously along with STEP "a" (P10):

      - STEP "g" (P70) of optimized control of temperature in a fermentation tank (0, D1) by means of acoustic emission techniques;
      - STEP "h" (P80) of optimized control of aeration or oxygenation in a fermentation tank (0, D1) by means of acoustic emission techniques.

- **STEP "b" (P20): Providing, in a controlled manner, by means of acoustic emission techniques, a fermentation tank (0, D1) with a gas mixture (O$_2$ + CO$_2$), allowing yeasts to perform respiration exclusively.**

The passage of O$_2$ to the fermentation tank (0, D1) is allowed or prevented to perform aeration or oxygenation by means of an aeration or oxygenation control valve (111), and the passage of CO$_2$ to said fermentation tank (0, D1) is allowed or prevented by means of an injection control valve (35). In this STEP "b" (P20), the naturally present or artificially inoculated yeast performs, by means of the supply of gas (O$_2$ and CO$_2$), respiration controlled by means of acoustic emission techniques in a fermentation tank (0) in which a device (D1) object of the invention has been implemented. STEP "b" is similar to STEP "a" but with the difference that a gas mixture containing O$_2$ instead of air is provided.
STEP "b" (P20) of respiration with O$_2$ comprises at least the following sub-steps:

- injecting O$_2$ in the fermentation tank (0, D1) (P21);
- suctioning CO$_2$ + injecting O$_2$ in the fermentation tank (0, D1) (P22);
- suctioning CO$_2$ + injecting O$_2$ and CO$_2$ in the fermentation tank (0, D1) (P23).

The following can be selected simultaneously along with STEP "b" (P20):

- STEP "g" (P70) of optimized control of temperature in a fermentation tank (0, D1) by means of acoustic emission techniques;
- STEP "h" (P80) of optimized control of aeration or oxygenation in a fermentation tank (0, D1) by means of acoustic emission techniques.
- **STEP "c" (P30): Providing a fermentation tank (0, D1) with a gas mixture (CO$_2$ + air), allowing yeasts to perform combined alcoholic fermentation and respiration monitored and controlled by means of acoustic emission techniques.**

The passage of air to the fermentation tank (0, D1) is allowed or prevented to perform aeration or oxygenation by means of an aeration or oxygenation control valve (111), and the passage of CO$_2$ to said fermentation tank (0, D1) is allowed or prevented by means of an injection control valve (35). In this STEP "c" (P30), the naturally present or artificially inoculated yeast performs, by means of the supply of gas (CO$_2$ and air), alcoholic fermentation monitored and controlled by means of acoustic emission techniques in a fermentation tank (0) in which a device (D1) object of the invention has been implemented. The CO$_2$ (gas) generated during alcoholic fermentation remains in dissolution until it is released once the fermentation must (liquid) becomes saturated, whereas ethanol (liquid) remains in dissolution, although a small fraction of ethanol is lost through evaporation. The CO$_2$ is suctioned for subsequent injection in the fermentation must for the purpose of achieving homogenization, avoiding the performance of pumping over. The aeration in this STEP "c" allows the yeast to also perform respiration for the purpose of facilitating its growth and reproduction by providing it with the required air (of which the yeast will use oxygen), such that a part of the sugars in the must undergoes respiration instead of fermentation. STEP "c" differs from STEP "a" **in that** in STEP "a" respiration is performed continuously for some time, whereas in this STEP "c" respiration can be performed discontinuously or continuously together with fermentation, producing wines with more color that is fixed by the oxygen from aeration, in addition to achieving a greater disassociation of tannins and anthocyanins.
STEP "c" (P30) of alcoholic fermentation plus aeration is **characterized in that** it comprises at least the following sub-steps:

- injecting air in the fermentation tank (0, D1) (P11);
- suctioning CO$_2$ + injecting air in the fermentation tank (0, D1) (P12);
- suctioning CO$_2$ + injecting air and CO$_2$ in the fermentation tank (0, D1) (P13);
- suctioning CO$_2$ + injecting CO$_2$ in the fermentation tank (0, D1) (P31);
- suctioning CO$_2$ in the fermentation tank (0, D1) (P32).

The following can be selected simultaneously along with STEP "c" (P30):

- STEP "g" (P70) of optimized control of temperature in a fermentation tank (0, D1) by means of acoustic emission techniques;
- STEP "h" (P80) of optimized control of aeration or oxygenation in a fermentation tank (0, D1) by means of acoustic emission techniques.

**- STEP "d" (P40): Providing a fermentation tank (0, D1) with a gas mixture (CO$_2$ + O$_2$), allowing yeasts to perform combined alcoholic fermentation and respiration monitored and controlled by means of acoustic emission techniques.**

The passage of O$_2$ to the fermentation tank (0, D1) is allowed or prevented to perform aeration or oxygenation by means of an aeration or oxygenation control valve (111), and the passage of CO$_2$ to said fermentation tank (0, D1) is allowed or prevented by means of an injection control valve (35). In this STEP "d" (P40), the naturally present or artificially inoculated yeast mainly performs, by means of the supply of gas (CO$_2$ and O$_2$), alcoholic fermentation monitored and controlled by means of acoustic emission techniques in a fermentation tank (0) in which a device (D1) object of the invention has been implemented. STEP "d" is similar to STEP "c" but with the difference that a gas mixture containing O$_2$ instead of air is provided. The oxygenation of this STEP "d" allows the yeast to also perform respiration for the purpose of facilitating its growth and reproduction by providing it with the required oxygen, such that a part of the sugars in the must undergoes respiration instead of fermentation. STEP "d" differs from STEP "b" in that in STEP "b" respiration is performed continuously for some time, whereas in this STEP "d" respiration can be performed discontinuously or continuously together with fermentation, producing, like in the preceding step, wines with more color that is fixed by the oxygen from oxygenation, in addition to achieving a greater disassociation of tannins and anthocyanins.

STEP "d" (P40) of alcoholic fermentation plus oxygenation is **characterized in that** it comprises at least the following sub-steps:

- injecting O$_2$ in the fermentation tank (0, D1) (P13);
- suctioning CO$_2$ + injecting O$_2$ in the fermentation tank (0, D1) (P22);
- suctioning CO$_2$ + injecting O$_2$ and CO$_2$ in the fermentation tank (0, D1);
- suctioning CO$_2$ + injecting CO$_2$ in the fermentation tank (0, D1) (P31);
- suctioning CO$_2$ in the fermentation tank (0, D1) (P32).

The following can be selected simultaneously along with STEP "d" (P40):

- STEP "g" (P70) of optimized control of temperature in a fermentation tank (0, D1) by means of acoustic emission techniques;
- STEP "h" (P80) of optimized control of aeration or oxygenation in a fermentation tank (0, D1) by means of acoustic emission techniques.

**- STEP "g" (P70): Monitoring and controlling the temperature in a fermentation tank (0, D1) by means of acoustic emission techniques.**

Since alcoholic fermentation is an exothermic reaction, the heat generated during the process of fermentation must be removed in order to maintain the optimal temperature. To that end, a cooling system of any of the state of the art is used which, by means of a cooling control valve (113), allows or prevents the passage of the liquid coolant to the jackets (05) of the fermentation tank (0, D1). The acoustic emission (ACO$_2$) caused by the CO$_2$ generated during respiration and alcoholic fermentation is measured and the acoustic emission speed (dACO$_2$/dt) is calculated for the early detection of the fermentation speed (g of CO$_2$/l/h) (amount, by weight or by volume, of CO$_2$ produced per unit of time), and therefore the activity of the yeasts. The analog signal of the omnidirectional hydrophone sensor (20) is collected in the slave panel (41), being sent to the main control panel (40) and reaching the programmable logic controller (401) where it is internally converted to a digitalized signal, first being processed in a calibration module (P101) of the program (P0) to obtain the basic amplitude, frequency, and spectral descriptors thereof by applying fast Fourier transform (FFT); with the correct frequency range, the digitalized signal passes to the filtration module (P102) where a digital filter is applied to the signal to discriminate frequencies not related with the process of fermentation, finally obtaining the real-time value of the acoustic emission (ACO$_2$).

When the acoustic emission speed (dACO$_2$/dt), i.e., CO$_2$ production, exceeds a previously established limit ((ACO$_2$)setpoint), taking into account a preestablished hysteresis value ($\Delta$(ACO$_2$)), the cooling control valve (113) is opened to keep the temperature within compatible winemaking limits to stabilize alcoholic fermentation, according to the following simple conditional logic equation:

$$\text{"If" } 113 = 0 \text{ "AND" } T \geq (\text{Tsetpoint} + \Delta T) \text{ "O"}$$

$$(\text{Tsetpoint} - \Delta T) < T < (\text{Tsetpoint} + \Delta T) \text{ "AND" } \left( \frac{d ACO_2}{dt} \geq ((ACO_2)\text{setpoint} + \Delta(ACO_2)) \right)$$

$$\text{"then"} \{113 = 1\}$$

When the acoustic emission speed ($dACO_2/dt$), i.e., $CO_2$ production, is below a previously established limit (($ACO_2$)setpoint), taking into account a preestablished hysteresis value ($\Delta(ACO_2)$), the cooling control valve (113) is closed allowing the temperature to rise to within compatible winemaking limits in order to reactivate alcoholic fermentation, according to the following simple conditional logic equation:

$$\text{"If" } 113 = 1 \text{ "AND" } T \leq (\text{Tsetpoint} - \Delta T) \text{ "O"}$$

$$(\text{Tsetpoint} - \Delta T) < T < (\text{Tsetpoint} + \Delta T) \text{ "AND" } \left( \frac{d ACO_2}{dt} \leq ((ACO_2)\text{setpoint} - \Delta(ACO_2)) \right)$$

$$\text{"then"} \{113 = 0\}$$

**- STEP "h" (P80): Monitoring and controlling the aeration or oxygenation of a fermentation tank (0, D1) by means of acoustic emission techniques.**

The passage of air or oxygen to the fermentation tank (0, D1) is allowed or prevented to perform aeration or oxygenation by means of an aeration or oxygenation control valve (111). The acoustic emission ($ACO_2$) caused by the $CO_2$ generated during respiration and alcoholic fermentation is measured and the acoustic emission speed ($dACO_2/dt$) is calculated for the early detection of the fermentation speed (g of $CO_2/l/h$), and therefore the activity of the yeasts. The analog signal of the omnidirectional hydrophone sensor (20) is collected in the slave panel (41), being sent to the main control panel (40) and reaching the programmable logic controller (401) where it is internally converted to a digitalized signal, first being processed in a calibration module (P101) of the program (P0) to obtain the basic amplitude, frequency, and spectral descriptors thereof by applying fast Fourier transform (FFT); with the correct frequency range, the digitalized signal passes to the filtration module (P102) where a digital filter is applied to the signal to discriminate frequencies not related with the process of fermentation, finally obtaining the real-time value of the acoustic emission ($ACO_2$),

When the acoustic emission speed ($dACO_2/dt$), i.e., $CO_2$ production, is below a previously established limit (($ACO_2$)setpoint), taking into account a preestablished hysteresis value ($\Delta(ACO_2)$), the aeration or oxygenation control valve (111) is opened in order to reactivate the activity of the yeasts, according to the following simple conditional logic equation:

$$\text{"If" } 111 = 0 \text{ "AND" } \left( \frac{d ACO_2}{dt} \leq ( (ACO_2)\text{setpoint} - \Delta(ACO_2)) \right)$$

$$\text{"then"} \{111 = 1\}$$

When the acoustic emission speed ($dACO_2/dt$), i.e., $CO_2$ production, exceeds a previously established limit (($ACO_2$)setpoint), taking into account a preestablished hysteresis value ($\Delta(ACO_2)$), the aeration or oxygenation control valve (111) is closed in order to deactivate the excessive activity of the yeasts, according to the following simple conditional logic equation:

$$\text{"If" } 111 = 1 \text{ "AND" } \left( \frac{d ACO_2}{dt} \geq ( (ACO_2)\text{setpoint} + \Delta(ACO_2)) \right)$$

$$\text{"then"} \{111 = 0\}$$

4. Method according to claim 3, **characterized by** the fact that noise from aeration, oxygenation, $CO_2$ injection, cooling water recirculation through the jackets, and any other desired noise is eliminated to obtain the acoustic emission

(ACO$_2$); to that end, before the start of alcoholic fermentation the acoustic emission of aeration (AAera), of oxygenation (AO$_2$), of CO$_2$ injection (ALCO$_2$), of cooling (AR), and of any other desired noise (AO) is recorded; then, noise is eliminated from the acoustic emission measured in the fermentation must (AMF) by means of the following equation:

$$ACO_2 = AM - AAera - AO_2 - AICO_2 - AR - AO$$

since the PLC program (PC), installed in the PLC (401), has at all times the state of the aeration, oxygenation, CO$_2$ injection, and cooling sub-steps so that in the case where one or more of them are activated, the corresponding noise from the active sub-steps can be subtracted by means of the preceding equation; that which is indicated above is performed if a noise emitting source is detected for the purpose of obtaining only the acoustic emission (ACO$_2$) caused by the CO$_2$ generated during respiration and alcoholic fermentation.

5. The method according to one of claims 3 to 4, **characterized by** the fact that the maximum aeration conditions which maintain the level of dissolved oxygen close to zero from the start and the levels of acetic acid at the end of the process at 0.3 g/l are:

   *- Airflow (l/h) = 5 volume (l) of fermentation must;*
   *- Time (h) = 48, counted in a continuous regimen or accumulated in a discontinuous regimen.*

6. Method according to one of claims 3 to 4, **characterized by** the fact that the maximum oxygenation conditions which maintain the level of dissolved oxygen close to zero from the start and the levels of acetic acid at the end of the process at 0.3 g/l are:

   *- Oxygen flow (l/h) = 1·volume (l) of fermentation must;*
   *- Time (h) = 48, counted in a continuous regimen or accumulated in a discontinuous regimen.*

7. Method according to claim 3, **characterized by** the fact that the calculation error of the density (D) of the fermentation must is obtained from measurements given by the pressure sensor (10) combined with the pressure sensors (13, 15) by means of an initial calibration before starting alcoholic fermentation which consists of performing a first measurement by means of the three pressure sensors (13, 15, and 10) when there is still no CO$_2$ dissolved in the fermentation must, an error-free measurement, and a second measurement by injecting CO$_2$, opening the injection control valve (35) of the gas processing and storage subsystem (3) until the pressure sensor (10) indicates the same CO$_2$ pressure as the working pressure marked by the inerting control valve (32), a measurement with error; the error produced in the pressure sensors (13, 15) for said CO$_2$ pressure is obtained with both measurements, subtracting the value of the measurement with error from the error-free measurement.

# FIG.01

# FIG.02

# FIG.03

# FIG.04

# FIG.05

# FIG.06

# FIG.07

FIG.08

# FIG.09

# FIG.10

# FIG.11

# FIG.12

# FIG.13

# FIG.14

# FIG.15

## FIG.16

FIG.17

HW=0K

START

STEP a)
RESPIRATION
WITH AIR

P10

FIG.18

Pa
Pa*

S
Q
R

STEP b)
RESPIRATION
WITH O₂

P20

FIG.19

Pb
Pb*

S
Q
R

STEP c)
ALC. FERM.
+AERATION

P30

FIG.20

Pc
Pc*

S
Q
R

STEP d)
ALC. FERM.
+ OXYGENA-
TION

P40

FIG.21

Pd
Pd*

S
Q
R

STEP e)
INERTING
WITH N₂

P50

Pe
Pe*

S
Q
R

STEP f)
NORMAL
STOP

P60

Pf
Pf*

S
Q
R

START

STEP g)
OPT.
CONTROL
TEMP.

P70

FIG.22

ST

STEP g)
OPT. CONTROL
AIR/O₂

P80

FIG.23

A/O

STEP j)
EMERGENCY
STOP

P90

PE

Pa*=Pb+Pc+Pd+Pe+Pf;Pb*=Pa+Pc+Pd+Pe+Pf;Pc*=Pa+Pb+Pd+Pe+Pf;Pd*=Pa+Pb+Pc+Pe+Pf; Pe*=Pa+Pb+Pc+Pd+Pf; Pf*=Pa+Pb+Pc+Pd+Pe.

# FIG.18

EP 4 324 903 A1

P20

HW=1

STEP b) RESPIRATION WITH $O_2$

f18<fsetpoint

(f18<fsetpoint) · (p10≥p10max)

(f18<fsetpoint) · (p10≥p10max) · (111on=1) · (p316>p316min)

FIG.13

FIG.14

FIG.15

P21

P22

P23

INJ. $O_2$ IN FERM. TANK (0, D1)

| 3131=1 |
| 111=1 |

SUC. $CO_2$ + INJ. $O_2$ IN FERM. TANK (0, D1)

| 3101=1 |
| 3111=1 |
| 3131=1 |
| 111=1 |
| 31=1 |

SUC. $CO_2$ + INJ. $O_2$ & $CO_2$ IN FERM. TANK (0, D1)

| 3101=1 |
| 3111=1 |
| 3131=1 |
| 111=1 |
| 31=1 |
| 35=1 |

p10≥p10max

(f18≥fsetpoint)+(p10≤p10min)

(f18≥fsetpoint)+(p10≤p10min) +(111on=0)+(p316≤p316min)

FIG.19

FIG.20

EP 4 324 903 A1

FIG.21

STEP d) ALCOHOLIC FERMENTATION + OXYGENATION

# FIG.22

STEP g) OPT. CONTROL OF TEMPERATURE

P70

$HW=1$

$(T \geq Tsetpoint+\Delta T)+$
$(Tsetpoint-\Delta T) < T < (Tsetpoint+\Delta T)\cdot$
$dACO_2/dt \geq (ACO_2)setpoint+\Delta(ACO_2)$

$(T \leq Tsetpoint-\Delta T)+$
$(Tsetpoint-\Delta T) < T < (Tsetpoint+\Delta T)\cdot$
$dACO_2/dt \leq (ACO_2)setpoint-\Delta(ACO_2)$

$113=0$

$113=1$

T

T

1

1

# FIG.23

STEP h) OPT. CONTROL OF AERATION OR OXYGENATION

P80

$dACO_2/dt \leq (ACO_2)setpoint - \Delta(ACO_2)$

$dACO_2/dt \geq (ACO_2)setpoint + \Delta(ACO_2)$

Hw=1

$O_2$   111=1

$O_2$   111=0

# INTERNATIONAL SEARCH REPORT

| International application No. |
| --- |
| PCT/ES2021/070247 |

**A. CLASSIFICATION OF SUBJECT MATTER**

*C12G1/02* (2006.01)
*B01F13/00* (2006.01)
According to International Patent Classification (IPC) or to both national classification and IPC

**B. FIELDS SEARCHED**

Minimum documentation searched (classification system followed by classification symbols)
C12G, B01F

Documentation searched other than minimum documentation to the extent that such documents are included in the fields searched

Electronic data base consulted during the international search (name of data base and, where practicable, search terms used)

EPODOC, INVENES

**C. DOCUMENTS CONSIDERED TO BE RELEVANT**

| Category* | Citation of document, with indication, where appropriate, of the relevant passages | Relevant to claim No. |
| --- | --- | --- |
| A | EP 1270716 A1 (LICHT STEPHAN DIPL-ING   ET AL.) 02/01/2003, The whole document. | 1-7 |
| A | ES 2396676 A1 (MAQUINAS Y HERRAMIENTAS LA RIOJA S L) 25/02/2013, The whole document. | 1-7 |
| A | ES 2241985T T3 (BOCK HERMANN) 01/11/2005, The whole document. | 1 |
| A | EP 2692846 A2 (NOFORM S R L) 05/02/2014, The whole document. | 1 |

☐ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
| --- | --- |
| 20/12/2021 | **(22/12/2021)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>V. Anguiano Mañero<br><br><br>Telephone No. 91 3495538 |

Form PCT/ISA/210 (second sheet) (January 2015)

## INTERNATIONAL SEARCH REPORT

Information on patent family members

International application No.

PCT/ES2021/070247

| Patent document cited in the search report | Publication date | Patent family member(s) | Publication date |
|---|---|---|---|
| EP1270716 A1 | 02.01.2003 | DE10131158 A1 | 16.01.2003 |
| ES2396676 A1 | 25.02.2013 | ES2681368T T3<br>WO2014072562 A1<br>EP2918666 A1<br>EP2918666 A4 | 12.09.2018<br>15.05.2014<br>16.09.2015<br>07.09.2016 |
| ES2241985T T3 | 01.11.2005 | EP1329254 A1<br>EP1329254 B1<br>AT293009T T<br>AT5698U U1 | 23.07.2003<br>13.04.2005<br>15.04.2005<br>25.10.2002 |
| EP2692846 A2 | 05.02.2014 | ITTV20120149 A1 | 31.01.2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- ES 2396676 B1
- EP 1270716 A1
- CA 2011297 A1

- ES 2330242 T3
- ES 2241457 B1

### Non-patent literature cited in the description

- **AGUILAR, R. et al.** Robust PI2 controller for continuous bioreactors. Process. *Biochemistry,* 2001, vol. 36, 1007-1013
- **BOVÉE J.P ; BLOUIN L ; MARIÓN, J.M. ; STREHAIANO, P.** In Actualités Enologiques. 1990, vol. 90, 281-286
- **CENZANO, JM. ; VICENTE, AM. ; TEJERO, GM.** Análisis de vinos, mostos y alcoholes. *Antonio Madrid Vicente Ediciones and Ediciones Mundi-Prensa,* 2003
- **FLANZY, C.** Enologia: fundamentos cientificos y tecnológicos, 2a. *A. VICENTE EDICIONES and EDICIONES MUNDI-PRENSA,* 2003
- **GONZÁLEZ R. ; QUIRÓS M. ; MORALES R.** Yeast respiration of sugars by non-Saccharomyces yeast species: a promising and barely explored approach to lowering alcohol content of wines. *Trends Food Sci Technol,* 2013, vol. 29, 55-61
- **GRENIER P. ; FEUILLOLEY, P. ; SABLAYROLLES, J.M.** In Actualités Enologiques. 1990, vol. 89, 521-526
- **HIDALGO, J.** Tratado de Enologia. *Tomo 1, 2a. Mundi-Prensa,* 2011
- **HODGE, D. ; NAZMUL, M.** Nonlinear MPC for recombinant ZM fedbatch ethanol fermentation. *IFAC World Congress,* 2002

- **MORALES, P. et al.** Levaduras no Saccharomyces como herramientas para controlar el grado alcohólico de los vinos: importancia del oxigeno y la respiración. Instituto de Ciencias de la Vid y del Vino. *CSIC - Universidad de La Rioja - La Rioja Government,* 2015, http://www.acenologia.com/cienciaytecnologia/no_saccharomyces_y_oxigeno_cie nc0515.htm
- *International Organization of Vine and Wine.,* 2015, http://www.oiv.int/es/la-organizacion-internacional-de-la-vina-y-el-vino
- **ONDER, M. et al.** Neural Network Assisted Nonlinear Controller for a Bioreactor Bogazici. Istanbul University, 1998
- The Microbiology of Wine and Vinifications. **RIBÉREAU-GAYON, P. ; DUBOURDIEU, D. ; DONÉCHE, B. ; LONVAUD, A.** Handbook of Enology. John Wiley & Sons, 2006, vol. 1
- The Chemistry of Wine Stabilization and Treatments. **RIBÉREAU-GAYON, P. ; GLORIES, Y. ; MAUJEAN, A. ; DUBOURDIEU, D.** Handbook of Enology. John Wiley & Sons, 2006, vol. 2
- **SING, K. ; XIAO, D.** Simple procedure for operating a class of continuous fermentation process at the optimal steady state production. *Biochemical Engineering Journal,* 1997, vol. 1